# EUROPÄISCHE PATENTSCHRIFT

(11) **EP 1 545 230 B1**
(45) Veröffentlichungstag und Bekanntmachung des Hinweises auf die Patenterteilung: **16.05.2007**
(21) Anmeldenummer: 03748024.1
(22) Anmeldetag: 13.09.2003
(51) Int. Cl.: A23G 4/06, A61K 9/00

(54) **UMHÜLLTER KAUGUMMI**
COATED CHEWING GUM
GOMME A MACHER ENROBEE

(30) Priorität: 23.09.2002 DE 10248632
(43) Veröffentlichungstag der Anmeldung: 29.06.2005
(73) Patentinhaber: Sustech GmbH & Co. KG, 64287 Darmstadt (DE); Südzucker Aktiengesellschaft Mannheim/Ochsenfurt, 68165 Mannheim (DE)
(72) Erfinder: SCHECHNER, Gallus, 64285 Darmstadt (DE); BRAUNBARTH, Carola, 64380 Rossdorf (DE); POTH, Tilo, 69469 Weinheim (DE); FRANKE, Holger, 64462 Ginsheim (DE); GUDERJAHN, Lutz, 67591 Offstein (DE); KOWALCZYK, Jörg, 67304 Eisenberg (DE)
(74) Vertreter: Ulbrich, Carola
(86) Internationale Anmeldenummer: PCT/EP2003/010213
(87) Internationale Veröffentlichungsnummer: WO 2004/028262

(56) Entgegenhaltungen:
- EP-A- 0 091 611
- EP-A- 0 263 224
- EP-A- 0 414 932
- US-A- 5 980 955
- US-A1- 2001 021 373
- US-A1- 2001 021 403

## Beschreibung

Die Erfindung betrifft einen Kaugummi, der von mindestens einer Schicht umhüllt ist, wobei diese Schicht schwer wasserlösliches Calciumsalz und/oder dessen Komposite umfasst, wobei das schwer warserlösliche Calciumsalz eine Teilchengröße kleiner 1000 nm aufweist.

Speisereste, die nach dem Essen im Mund verbleiben, sind eine der Hauptursachen für das Auftreten von Karies. In den meisten Fällen wird auf das Putzen der Zähne nach dem Essen einfach verzichtet. Insbesondere der darin enthaltene Zucker dient als Nährstoff für Bakterien der Mundhöhle, die einerseits durch bakterielle Abbauprodukte (insbesondere organische Säuren wie Milch-, Ameisen- oder Essigsäure) und andererseits durch eine vermehrte Plaquebildung für die Entstehung von Karies verantwortlich sind.

Das Kauen von Kaugummi nach dem Essen soll der Bildung von kariesfördernden bakteriellen Abbauprodukten entgegenwirken. Dazu werden in diesen Kaugummis sogenannte Zuckeraustauschstoffe, insbesondere Zuckeralkohole wie Sorbit, Isomalt und Xylit eingesetzt. Zwar wird durch den erhöhten Speichelfluss die Bildung von kariesverursachenden Säuren verhindert oder zumindest vermindert, die allgemeine Zahngesundheit können diese Kaugummis aber nur in begrenztem Maße verbessern.

Der Zahnschmelz sowie das Stützgewebe der Knochen bestehen überwiegend aus dem Mineral Hydroxylapatit. Die Zugabe von Calcium- und/oder Phosphatsalzen zu Kaugummis soll dazu dienen, die Remineralisierung des Zahnschmelzes zu verbessern.

Das Patent US 5,980,955 beschreibt eine Umhüllung von Kaugummis mit schwer löslichen Salzen, z.B. Calciumphosphaten, zur Verbesserung der Oberflächenbeschaffenheit der Kaugummis.

Die Patentanmeldung US 2001/0021403 und US 2001/0021373 offenbaren Verfahren zur Herstellung von mit einer säurebindenden Komponente umhüllten Kaugummis. Als säurebindende Komponenten können Calciumcarbonate oder Calciumhydroxid eingesetzt werden.

Nachteilig an diesen Zusammensetzungen ist, dass durch die Zugabe von herkömmlichen gemahlenen, mikrokristallinen Calcium- und/oder Phosphatsalzen keine ausreichende Remineralisierung des Zahnmaterials zu erhalten ist.

Die Aufgabe der vorliegenden Erfindung ist es daher, alternativ einen Kaugummi bereitzustellen, der neben einem guten Geschmack zusätzlich einen positiven Nutzen für die Zahngesundheit während und/oder kurz nach seinem Genuss aufweist.

Diese Aufgabe wird gelöst durch einen Kaugummi, der von mindestens einer Schicht umhüllt ist, wobei diese Schicht schwer wasserlösliches Calciumsalz und/oder dessen Komposite umfasst und wobei das schwer wasserlösliche Calciumsalz eine Teilchengröße kleiner 1000 nm aufweist.

Der erfindungsgemäße Kaugummi besteht aus Zuckerarten und/oder Zuckeralkoholen, Intensivsüßstoffen, Aromen, anderen geruch- und geschmack- oder konsistenzgebenden Zutaten, Farbstoffen sowie einer wasserunlöslichen, beim Kauen plastisch werdenden Kaumasse. Daneben können die Kaugummis auch Trennmittel (wie beispielsweise Talkum) enthalten.

Kaumassen sind Gemische aus konsistenzgebenden Stoffen, den natürlichen Gummen, das sind erstarrte Säfte (Exudate) aus tropischen Pflanzen wie Chicle, Gummi arabicum, Guttapercha, Karayagummi und Traganth, Kautschuk und den thermoplastischen Kunststoffen Butadien-Styrol-Copolymerisate, Isobutylen-Isopren-Copolymerisate, Polyethylen, Polyisobutylen, Polyvinylester der unverzweigten Fettsäuren von C₂ bis C₁₈ und Polyvinylether.

Als Plastifikatoren werden Harze und Balsame eingesetzt. Zu den natürlichen Stoffen zählen Benzoeharz, Dammarharz, Kolophonium, Mastix, Myrrhe, Olibanum, Perubalsam, Sandarrak, Schellack und Tolubalsam, zu den synthetischen Cumaron-Inden-Harz, Glycerin-Pentaerythritester der Harzsäuren des Kolophoniums und deren Hydrierungsprodukte.

Zur Beeinflussung der Elastizität finden Paraffine (natürliche und synthetische) sowie Wachse Verwendung. Bei den Wachsen gibt es solche aus dem pflanzlichen Bereich wie Carnabauwachs und solche tierischen Ursprungs wie Bienenwachs oder Wollwachs, daneben solche aus dem mineralischen Bereich wie mikrokistalline Wachse, wie auch chemisch modifizierte oder synthetische Wachse. Als Weichmacher dienen Emulgatoren (z.B. Lecithine oder Mono- und Diglyceride von Speisefettsäuren) und Ester wie Glycerinacetat sowie auch Glycerin.

Zur Regulierung der Kaumassenkonsistenz werden pflanzliche Hydrokolloide wie Agar-Agar, Alginsäure und Alginate, Guarkernmehl, Johannisbrotkernmehl oder Pektin zugesetzt. Zur gezielten Einstellung der Kaueigenschaften von Kaumassen werden Füllstoffe eingesetzt, das sind Carbonate von Calcium oder Magnesium, Oxide, beispielsweise Aluminiumoxid, Kieselsäure und Silicate von Calcium oder Magnesium. Stearinsäure und ihre Calcium- und Magnesiumsalze werden zur Verminderung des Haftvermögens der Kaumasse am Zahnschmelz eingesetzt.

Bevor die übrigen, für die Herstellung von Kaugummi rezepturgemäß erforderlichen Zutaten untergemischt werden, ist es erforderlich, die Kaumasse, die etwa 20-35% (mindestens aber 15%) des fertigen Kaugummis ausmacht, auf 50-60°C zu erwärmen.

Durch die ausgeführte Kaubewegung fördert der Kaugummi den Speichelfluss. Die kariesverursachenden Säuren werden verdünnt und so auf natürliche Art die Gesundheit des Mundraums unterstützt.

Als in Wasser schwerlösliches Calciumsalz sollen solche Salze verstanden werden, die bei 20°C zu weniger als 0,1 Gew.-% (1 g/l) in Wasser löslich sind. Solche geeigneten Salze sind z.B. Calciumhydroxyphosphat (Ca₅[OH(PO₄)₃]) bzw. Hydroxylapatit, Calciumfluorphosphat (Ca₅[F(PO₄)₃]) bzw. Fluorapatit, fluordotierter Hydroxylapatit der Zusammensetzung Ca₅(PO₄)₃(OH,F) und Calciumfluorid (CaF₂) bzw. Fluorit oder Flußspat sowie andere Calciumphosphate wie Di-, Tri- oder Tetracalciumphosphat (Ca₂P₂O₇, Ca₃(PO₄)₂, Ca₄P₂O₉, Oxyapatit (Ca₁₀(PO₄)₆O) oder nichtstöchiometrischer Hydroxylapatit (Ca_{5-1/2 (x+y)} (PO₄)₃ₓ (HPO₄)ₓ(OH)_{1-y}). Geeignet sind ebenfalls carbonathaltiger nichtstöchiometrischer Apatit (z.B. Ca_{5-1/2 (x+y+z)} (PO₄)_{3-x-z} (HPO₄)ₓ (CO₃)_{z}(OH)_{1-y}), Calciumhydrogenphosphat (z.B. CaH(PO₄)*2 H₂O) und Octacalciumphosphat (z.B. Ca₈H₂(PO₄)₆*5 H₂O).

Als mineralisierender Wirkstoff eignet sich bevorzugt ein feinteiliges, in Wasser schwerlösliches Calciumsalz, welches ausgewählt ist aus Hydroxylapatit, carbonathaltigem nichtstöchiometrischem Apatit, Fluorapatit, Fluor-dotiertem Hydroxylapatit und Gemische davon. Diese Calciumsalze können sich am besten am Zahnmaterial anlagern und eine Mineralisierung desselben bewirken.

Unter Kompositmaterialien werden Verbundstoffe verstanden, die ein schwer wasserlösliches Calciumsalz und sowie weitere Komponenten umfassen und mikroskopisch heterogene, makroskopisch aber homogen erscheinende Aggregate darstellen.

Die feinteiligen Calciumsalze oder die in den Kompositmaterialien vorliegenden feinteiligen Calciumsalz-Primärteilchen können auch von einem oder mehreren Oberflächenmodifikationsmitteln umhüllt sein.

Dadurch kann beispielsweise die Herstellung von Kompositmaterialien in solchen Fällen erleichtert werden, bei welchen sich die nanopartikulären Calciumsalze schwer dispergieren lassen. Das Oberflächenmodifikationsmittel wird an die Oberfläche der Nanopartikel adsorbiert und verändert sie dergestalt, dass die Dispergierbarkeit des Calciumsalzes zunimmt und die Agglomeration der Nanopartikel verhindert wird.

Darüber hinaus kann durch eine Oberflächenmodifikation die Struktur der Kompositmaterialien sowie die Beladung von weiteren Komponenten mit dem nanopartikulären Calciumsalz beeinflusst werden. Auf diese Weise ist es bei der Anwendung der Kompositmaterialien in Mineralisationsprozessen möglich, Einfluss auf den Verlauf und die Geschwindigkeit des Mineralisationsprozesses zu nehmen.

Unter Oberflächenmodifikationsmitteln sind Stoffe zu verstehen, welche an der Oberfläche der feinteiligen Partikel physikalisch anhaften, mit diesen jedoch nicht chemisch reagieren. Die einzelnen an der Oberfläche adsorbierten oder gebundenen Moleküle der Oberflächenmodifikationsmittel sind im wesentlichen frei von intermolekularen Bindungen untereinander. Unter Oberflächenmodifikationsmitteln sind insbesondere Dispergiermittel zu verstehen. Dispergiermittel sind dem Fachmann auch unter den Begriffen Tenside und Schutzkolloide bekannt. Geeignete Tenside oder polymere Schutzkolloide können der deutschen Patentanmeldung DE 198 58 662 A1 entnommen werden.

Die Herstellung der erfindungsgemäßen Kompositmaterialien, in welchen die Primärpartikel der Calciumsalze oberflächenmodifiziert sind, kann nach analogen Fällungsverfahren wie vorstehend beschrieben erfolgen, wobei jedoch die Fällung der nanopartikulären Calciumsalze oder der Kompositmaterialien in Gegenwart eines oder mehrerer Oberflächenmodifikationsmittel erfolgt.

Die den Kaugummi umhüllende Schicht, die das schwer wasserlösliche Calciumsalz umfasst, führt vorteilhafterweise dazu, dass die Freisetzung des Calciumsalzes einfacher erfolgen kann als bei der direkten Einarbeitung der Salze in die Kaugummimasse, bei der die eingearbeiteten Calciumsalze stark mit der klebrigen Matrix der Kaumasse verhaftet bleiben. Die den Kaugummi umhüllende Schicht löst sich beim Kauen im Mund sehr schnell auf und kann so die notwendige Wirkstoffmenge im Mund verfügbar machen, die vorteilhafterweise eine effektive Mineralisierung der Zähne gewährleistet. Durch die Zugabe der Calciumsalze und/oder Kompositen davon wird der Crunch, also die Knusprigkeit der Kaugummis nicht beeinflusst.

Gemäß einer besonderen Ausführungsform enthält die den erfindungsgemäßen Kaugummi umhüllende Schicht Zucker und/oder Zuckeralkohole.

Vorteilhafterweise löst sich die Zucker und/oder Zuckeralkohole enthaltene Schicht besonders schnell im Mund auf. Sie kann neben dem süßen Geschmackserlebnis auch besonders gut auf einen Kaugummi-Kern aufgebracht werden.

Trotz der zum Teil zahnschädigenden Inhaltsstoffe (Zucker) führt der Konsum der erfindungsgemäßen Kaugummis neben dem Genusserlebnis zur Zahnpflege und Zahnschonung sowie darüber hinaus zur Mineralisierung des Zahnschmelzes und/oder des Dentins. Auf die, nicht immer nach dem Essen mögliche, aber zur Gesunderhaltung der Zähne bisher nötige Zahnpflege, üblicherweise mit Zahnbürste, Zahnpasta und/oder Mundwasser, kann so ohne Schaden für die Zähne verzichtet werden.

Der erfindungsgemäße Zusatz von schwer wasserlöslichen Calciumsalzen und/oder deren Kompositen in zuckeraustauschstoffhaltigen Kaugummis bewirkt eine Mineralisierung der Zähne während und/oder nach dem Genuss des Kaugummis und trägt somit besonders zum Erhalt gesunder Zähne bei. Vorteilhafterweise eignen sich die Zuckeralkohole aufgrund ihrer physiko-chemischen Eigenschaften besonders zur Herstellung von dünnen Schichten, vor allem im Dragierverfahren. Besonders bevorzugt ist der Einsatz von Isomalt in der umhüllenden Schicht, da dieser Zuckeralkohol eine vergleichsweise hohe Glasübergangstemperatur besitzt, die die Verarbeitung besonders erleichtert.

Die den Kaugummi umhüllende Schicht kann auf unterschiedliche Weisen, z.B. durch mehrfaches Tauchen des Kaugummi-Kerns in eine entsprechende Lösung und/oder Dispersion, erzeugt werden.

Nach einer bevorzugten Ausführungsform der Erfindung ist die den Kaugummi umhüllende Schicht eine dragierte Schicht, d.h. die Schicht wird im Dragierverfahren auf den Kaugummi aufgebracht. Die Dragierschicht (Decke) besteht aus einer glatten oder gekrausten, mit Zuckerarten und/oder Zuckeralkoholen, Schokoladenarten und/oder anderen Glasuren, die um einen flüssigen, weichen oder festen Kern mittels des Dragierverfahrens aufgebracht wird.

Beim Dragierverfahren wird bspw. eine gesättigte Zuckerlösung feinverteilt aus einer Düse auf den in Dragierkesseln rotierenden Kern eingesprüht. Der Zucker kristallisiert durch die gleichzeitig eingeblasene warme Luft aus und bildet nach und nach viele dünne Schichten um den Kern. Enthält die Zuckerschicht keine Restfeuchte, wird es als Hartdragee bezeichnet, bei Weichdragees können dagegen etwa 6 bis 12, insbesondere 8 bis 10 Gew.-% Restfeuchte gegeben sein. Dragees werden äußerlich häufig mit einer dünnen Trenn- und Glanzschicht versehen, wobei die Glanzschicht durch Behandlung mit wachsartigen Stoffen, wie bspw. Carnaubawachs, entsteht. Insbesondere werden die Beschaffenheit beeinflussende Stoffe, wie z.B. Stärke sowie färbende, geruch- und geschmackgebende Stoffe eingesetzt.

Das in der umhüllenden Schicht enthaltene wenig bzw. schwer wasserlösliche Calciumsalz weist eine Teilchengröße bzw. Teilchenfeinheit kleiner 1000 nm auf. Als Teilchenfeinheit soll hier der Durchmesser der Teilchen in Richtung ihrer größten Längenausdehnung verstanden werden. Die mittlere Teilchenfeinheit bezieht sich auf einen volumengemittelten Wert. Die erfindungsgemäßen Nanopartikel besitzen ein größeres Oberflächen/Volumenverhältnis als die mikrokristallinen Teilchen und zeichnen sich durch eine höhere Reaktivität im Vergleich zu diesen aus. Sie können daher besser zur Remineralisierung von entmineralisiertem Zahnmaterial eingesetzt werden. Unter Remineralisierung ist in diesem Zusammenhang die Wiedereinlagerung von Ionen in Knochenmaterial, also das Auffüllen von Fehlstellen innerhalb des bestehenden Zahnhartgewebes, wie Schmelz und Dentin, zu verstehen.

Überraschenderweise wurde darüber hinaus gefunden, dass sich durch Zugabe des schwer wasserlöslichen Calciumsalzes und/oder seiner Komposite zusätzlich auf dem Zahn neue Schichten eines biomimetischen Materials bilden können. Dieses Material ist dem natürlichen Zahnhartgeweben chemisch und strukturell sehr ähnlich. Es werden daher nicht nur Fehlstellen innerhalb der Kristallstruktur ausgeglichen, wie es bei der Remineralisierung des Zahnmaterials geschieht, sondern auch neues, am Zahn anhaftendes, in seiner Nanostruktur dentinähnliches Material erzeugt. Diese Neuentstehung von biomimetischem Material wird im weiteren als Neomineralisierung bezeichnet. Im erfindungsgemäßen Zusammenhang sind von dem Begriff Mineralisierung sowohl die Re- als auch die Neomineralisierung umfasst.

Gemäß einer bevorzugten Ausführungsform weist das wenig bzw. schwer wasserlösliche Calciumsalz eine Teilchengröße bzw. Teilchenfeinheit von 5 bis 300 nm, insbesondere von 5 bis 100 nm auf. Ein Vorteil dieser besonders geringen Teilchengrößen bzw. -feinheiten besteht darin, dass diese Primärteilchen eine besonders effektive Remineralisierung der Zähne zeigen und darüber hinaus die Fähigkeit aufweisen, neue, neomineralisierte Schichten von dem Zahnhartgewebe sehr ähnlichem Material zu bilden.

Nach einer besonders bevorzugten Ausführungsform weisen die erfindungsgemäßen Calciumsalze eine längliche, insbesondere stäbchen- oder nadelähnliche Form auf. Dies hat den besonderen Vorteil, dass sie der Form der biologischen Apatite (z.B. Knochen- bzw. Dentinapatite) sehr ähnlich sind und daher eine besonders gute Fähigkeit zur Re- und Neomineralisierung aufweisen. Solche Calciumsalze lassen sich z.B. nach dem aus DE 198 58 662 A1 bekannten Verfahren in Form stäbchenförmiger Primärteilchen herstellen.

Gemäß einer bevorzugten Ausführungsform sind 0,001 bis 5 Gew.-% schwer wasserlösliches Calciumsalz und/oder dessen Komposite enthalten. Es ist bevorzugt, 0,01 bis 2 Gew.-% und insbesondere 0,1 bis 1 Gew.-% Calciumsalz und/oder dessen Komposite in der den Kaugummi umhüllenden Schicht einzusetzen.

Nach einer bevorzugten Ausführungsform enthält die umhüllende Schicht eines erfindungsgemäßen Kaugummis ein Komposit aus einem schwerlöslichen Calciumsalz mit einer Proteinkomponente.

Proteine können an die Oberfläche der Nanopartikel adsorbiert werden, wodurch ein Kompositmaterial aus Protein und schwer wasserlöslichem Calciumsalz entsteht. Insbesondere wird durch die adsorbierten Proteine auch eine Koagulation und Agglomeration der Calciumsalze verhindert und das Kristallwachstum verlangsamt. Bei der Mineralisation eines Zahns, und insbesondere bei der Neomineralisierung, ist es von großem Vorteil, wenn kein unkontrolliertes Kristallwachstum stattfindet, welches nur ein lockeres Kristallgefüge ausbilden könnte. Durch das Proteingerüst kann das Kristallwachstum gebremst und kontrolliert ablaufen. So wird ein besonders dichtes und festes Kristallgefüge gebildet.

Überraschenderweise wurde gefunden, dass die schwer wasserlöslichen Calciumsalze und insbesondere die Komposite aus schwer wasserlöslichem Calciumsalz mit Proteinen neben einer Remineralisation des Zahns auch in der Lage sind, das Ausmaß größerer Schäden im Zahndentin und/oder Zahnschmelz durch die Bildung vollständig neuer Kristalle zu verringern.

Bei der natürlichen Entstehung von Knochenmaterial wie z.B. des Zahnschmelzes und -dentins bewirkt eine Proteinmatrix die geordnete Anlagerung von Hydroylapatit im Zahn oder Knochen, die hauptsächlich aus Kollagen sowie anderen Proteinen besteht. Mit den Kompositen aus schwerlöslichem Calciumsalz und Proteinen verläuft die Neomineralisierung ähnlich wie die Biomineralisierung und führt damit zu einem besonders positiven Effekt auf die Zahngesundheit beim Genuss des erfindungsgemäßen Kaugummis.

Die in dem Komposit bevorzugt enthaltene Proteinkomponente ist insbesondere ausgewählt aus Proteinen, Proteinabbauprodukten und Derivaten von Proteinen oder Proteinabbauprodukten.

Als Proteine kommen dabei alle Proteine unabhängig von ihrer Herkunft in Frage, also sowohl tierische wie pflanzliche Proteine. Geeignete tierische Proteine sind z.B. Kollagen, Fibroin, Elastin, Keratin und Albumin. Geeignete pflanzliche Proteine sind z.B. Weizen- und Weizenkeimprodukte (Gluten), Reisprotein, Sojaprotein, Haferprotein, Erbsenprotein, Mandelprotein und Kartoffelprotein. Auch Einzellerproteine wie z.B. Hefeprotein oder Bakterienproteine sind geeignet.

Erfindungsgemäß bevorzugte Proteine sind tierische Produkte wie Kollagen und Keratin. Das Protein kann aber ebenfalls aus einer pflanzlichen oder marinen Quelle ausgewählt sein.

Als Proteinabbauprodukte werden solche Produkte verstanden, die durch hydrolytischen, oxidativen oder reduktiven Abbau von wasserunlöslichen Proteinen zu Oligo- und Polypeptidstrukturen mit niedrigerem Molekulargewicht und mit einer verbesserten Wasserlöslichkeit erhältlich sind.

Der hydrolytische Abbau wasserunlöslicher Proteine ist die wichtigste Abbaumethode, sie kann unter dem katalytischen Einfluss von Säuren, Alkalien oder von Enzymen erfolgen. Bevorzugt geeignet sind vor allem solche Proteinabbauprodukte, die nicht weiter abgebaut sind als zur Erlangung der Wasserlöslichkeit erforderlich.

Zu den wenig abgebauten Proteinhydrolysaten zählt beispielsweise die im Rahmen der vorliegenden Erfindung bevorzugte Gelatine, welche Molmassen im Bereich von 15000 bis 400000 D aufweisen kann. Gelatine ist ein Polypeptid, das vornehmlich durch Hydrolyse von Kollagen unter sauren oder alkalischen Bedingungen gewonnen wird. Besonders bevorzugt ist unter sauren oder stark sauren Bedingungen gewonnene Gelatine. Die Gelstärke oder Gelatine ist proportional zu ihrem Molekulargewicht, d.h. eine stärker hydrolysierte Gelatine ergibt eine niedriger viskose Lösung. Die Gelstärke der Gelatine wird in Bloom-Zahlen angegeben. Bei der enzymatischen Spaltung der Gelatine wird die Polymergröße stark erniedrigt, was zu sehr niedrigen Bloom-Zahlen führt.

Unter Derivaten von Proteinen und Proteinabbauprodukten werden chemisch modifizierte Proteine oder Proteinhydrolysate verstanden, die z.B. durch Acylierung freier Aminogruppen, durch Anlagerung von Ethylen- oder Propylenoxid und Hydroxyl-, Amino- oder Carboxylgruppen oder durch Alkylierung von Hydroxylgruppen des Proteins oder Proteinabbauproduktes oder eines Hydroxyalkylderivats davon, z.B. mit Epoxypropyl-trimethylammoniumchlorid oder 3-Chlor-2-hydroxypropyl-trimethylammoniumchlorid, erhältlich sind.

In einer besonders bevorzugten Ausführung ist die Proteinkomponente ausgewählt aus Gelatine, deren Hydrolysaten und Gemischen davon. Es sollte bevorzugt eine Proteinkomponente in einer Menge von wenigstens 1 Gew.-%, bevorzugt von 1 bis 50, insbesondere von 20 bis 40 Gew.-%, enthalten sein.

In den erfindungsgemäßen Kompositen liegen die Primärpartikel der Calciumsalze an das Gerüst der Proteinkomponente assoziiert vor. Der Anteil der Proteinkomponenten in solchen Kompositmaterialien liegt zwischen 0,1 und 50 Gew.-% bevorzugt jedoch zwischen 1,0 und 45 Gew.-%, insbesondere 20 bis 40 Gew.-% bezogen auf das Gewicht des Kompositmaterials.

Geeignet sind besonders Hydroxylapatit-Nanopartikel, die eine deutlich erkennbare kristalline Morphologie aufweisen, deren Teilchenfeinheit daher im Bereich von 5 bis 300 nm liegt. Ebenfalls geeignet sind Kompositmaterialien, bei denen die feinteiligen schwerlöslichen Calciumsalze mit Teilchenfeinheiten von 5 bis 300 nm zusammen mit feinteiligen Proteinen, Proteinhydrolysaten oder Derivaten davon eine räumliche Struktur derart bilden, dass die feinteiligen Calciumsalze der Proteinstruktur aufgelagert sind, diese quasi räumlich abbilden. Aus solchen bevorzugt geeigneten nanopartikulären Calciumsalzen und Proteinkomponenten bestehende Kompositmaterialien führen zu einer besonders guten Mineralisierung der Zähne beim Genuss des erfindungsgemäßen Kaugummis.

Besonders gut können sich schwer wasserlösliche Calciumsalze in Stäbchenform an die Proteinketten anlagern. Dies führt zu einem deutlich verbesserten Zusammenhalt des Kompositmaterials. Insbesondere geeignet sind dabei Primärpartikel mit einer Teilchenfeinheit von 5 bis 300 nm, und bevorzugt von 5 bis 100 nm, da diese besonders kleine Kristallite der Form biologischer Apatite sehr ähnlich und sich auch wegen der geringen Größe noch besser an die Proteinketten anlagern können. Diese Komposite führen dadurch zu einer besonders effektiven Mineralisierung von Zähnen.

Erfindungsgemäß geeignete kompositmaterialien können durch Fällung aus wässrigen Lösungen wasserlöslicher Calciumsalze mit wässrigen Lösungen wasserlöslicher Phosphat und/oder Fluoridsalze in Gegenwart von Proteinkomponenten nach unterschiedlichen Verfahren hergestellt werden, wie sie bereits in der deutschen Patentanmeldung DE 199 30 335 beschrieben sind.

Für die Herstellung der erfindungsgemäßen Kaugummis wird der Wirkstoff, also das feinteilige, in Wasser schwerlösliche Calciumsalz und/oder dessen Komposite, bevorzugt das Kompositmaterial aus dem schwerlöslichen Calciumsalz und einer Proteinkomponente, einfach zu einer Lösung und/oder Dispersion, aus der die Schicht hergestellt wird, zugegeben und eingerührt.

In einer bevorzugten Ausführungsform der vorliegenden Erfindung ist der erfindungsgemäße Kaugummi ein zuckerhaltiger Kaugummi. Im Zusammenhang mit der vorliegenden Erfindung werden unter "Zucker" beziehungsweise "Zucker-Arten" Produkte wie Saccharose, gereinigte kristalline Saccharose, beispielsweise in Form von raffiniertem Zucker, Raffinate, raffinierter Weißzucker, Weißzucker oder Halbweißzucker, wässrige Lösungen von Saccharose, beispielsweise in Form von Flüssigzucker, wässrige Lösungen von teilweise durch Hydrolyse invertierter Saccharose, beispielsweise Invertzucker, Sirup oder Invertflüssigzucker, Glucosesirup, getrockneter Glucosesirup, kristallwasserhaltige Dextrose, kristallwasserfreie Dextrose und andere Stärkeverzuckerungsprodukte sowie Trehalose, Trehalulose, Tagatose, Lactose, Maltose, Fructose, Leucrose, Isomaltulose (Palatinose), kondensierte Palatinose und hydrierte kondensierte Palatinose verstanden. Der erfindungsgemäße zuckerhaltige Kaugummi ist daher dadurch charakterisiert, dass entweder der Kaugummi selbst oder die umhüllende Schicht oder beide als Süßungsmittel Saccharose, Invertflüssigzucker, Invertzuckersirup, Glucose, Glucose-Sirup, Polydextrose, Trehalose, Trehalulose, Tagatose, Lactose, Maltose, Fructose, Leucrose, Isomaltulose (Palatinose), kondensierte Palatinose, hydrierte kondensierte Palatinose oder Gemische davon enthält. Der erfindungsgemäße zuckerhaltige Kaugummi kann neben den vorstehend genannten Zucker-Arten auch Zuckeraustauschstoffe, insbesondere Zuckeralkohole wie Lactit, Sorbit, Xylit, Mannit, Maltit, Erythrit, 6-O-α-D-Glucopyranosyl-D-sorbit (1,6-GPS), 1-O-α-D-Glucopyranosyl-D-sorbit (1,1-GPS), 1-O-α-D'-Glucopyranosyl-D-mannit (1,1-GPM), Maltit-Sirup, Sorbit-Sirup, Fructooligosaccharide oder Gemische davon sowie Gemische von Zuckeralkoholen und Zucker-Arten enthalten.

In einer weiteren bevorzugten Ausführungsform der Erfindung ist der erfindungsgemäße Kaugummi ein zuckerfreier Kaugummi. Im Zusammenhang mit der vorliegenden Erfindung wird unter einem "zuckerfreien Kaugummi" ein Kaugummi verstanden, bei dem sowohl der Kaugummi selbst als auch die umhüllende Schicht als Süßungsmittel keine der vorstehend genannten Zucker-Arten enthält, also weder Saccharose, Invertflüssigzucker, Invertzuckersirup, Dextrose, Glucose-Sirup, Trehalose, Trehalulose, Tagatose, Lactose, Maltose, Fructose, Leucrose, Isomaltulose (Palatinose), kondensierte Palatinose, hydrierte kondensierte Palatinose noch Gemische davon, sondern Zuckeraustauschstoffe. In bevorzugter Ausführungsform der Erfindung handelt es sich bei dem erfindungsgemäßen zuckerfreien Kaugummi um einen Kaugummi, der einen Maximalgehalt an den vorstehend genannten Zucker-Arten von 0,5 Gew.-%, bezogen auf das Trockengewicht, aufweist.

Der Begriff "Zuckeraustauschstoffe" umfasst alle Stoffe außer den vorstehend genannten Zucker-Arten, die zur Süßung von Lebensmitteln verwendet werden können. Der Begriff "Zuckeraustauschstoffe" umfasst insbesondere Substanzen wie hydrierte Mono- und Disaccharid-Zuckeralkohole, beispielsweise Lactit, Xylit, Sorbit, Mannit, Maltit, Erythrit, Isomalt, 1,6-GPS, 1,1-GPS, 1,1-GPM, Sorbit-Sirup, Maltit-Sirup, sowie Fructooligosaccharide. Vorzugsweise sind die erfindungsgemäßen zuckerfreien Kaugummis also dadurch charakterisiert, dass sowohl der Kaugummi selbst als auch die umhüllende Schicht als Süßungsmittel Lactose, Maltose, Fructose, Leucrose, Palatinose, kondensierte Palatinose, hydrierte kondensierte Palatinose, Fructooligosaccharide, Lactit, Sorbit, Xylit, Mannit, Maltit, Erythrit, 1,6-GPS, 1,1-GPS, 1,1-GPM, Sorbit-Sirup, Maltit-Sirup oder Gemische davon enthält. Erfindungsgemäß bevorzugt sind Zuckeralkohole wie Sorbit bzw. Sorbit-Sirup, Mannit, Xylit, Lactit, Maltit bzw. Maltit-Sirup, 1,1-GPS, 1,6-GPS, 1,1-GPM oder Gemische davon. Zuckeralkohole haben den Vorteil, dass sie pro 100 g weniger Kalorien enthalten und dass sie darüber hinaus durch Bakterien der Mundflora nur sehr langsam oder überhaupt nicht zu Säuren abgebaut werden, so dass sie nicht kariogen wirken.

Ein bevorzugt verwendetes Gemisch von 1,6-GPS und 1,1-GPM ist Isomalt, bei dem 1,6-GPS und 1,1-GPM in äquimolaren oder nahezu äquimolaren Mengen vorliegen. Erfindungsgemäß können in den erfindungsgemäßen Kaugummis, insbesondere zuckerfreien Kaugummis, sowohl im Kaugummi selbst als auch in der sie umhüllenden Schicht ebenfalls 1,6-GPS-angereicherte Gemische aus 1,6-GPS und 1,1-GPM mit einem 1,6-GPS-Anteil von 57 Gew.-% bis 99 Gew.-% und einem 1,1-GPM-Anteil von 43 Gew.-% bis 1 Gew.-%, 1,1-GPM-angereicherte Gemische aus 1,6-GPS und 1,1-GPM mit einem 1,6-GPS-Anteil von 1 Gew.-% bis 43 Gew.-% und einem 1,1-GPM-Anteil von 57 Gew.-% bis 99 Gew.-% sowie Gemische aus 1,6-GPS, 1,1-GPS und 1,1-GPM als Süßungsmittel eingesetzt werden. 1,6-GPS-angereicherte Gemische und 1,1-GPM-angereicherte Gemische aus 1,6-GPS und 1,1-GPM sind in der DE 195 32 396 C2 offenbart. 1,1-GPS-haltige Gemische von 1,6-GPS und 1,1-GPM sind beispielsweise in der EP 0 625 578 B1 offenbart.

Ein weiteres erfindungsgemäß bevorzugtes Gemisch, das in den erfindungsgemäßen Kaugummis, insbesondere zuckerfreien Kaugummis, eingesetzt werden kann, ist ein Sirup mit einer Trockensubstanz von 60 bis 80 %, bestehend aus einem Gemisch von hydiertem Stärkehydrolysatsirup und Isomalt-Pulver oder Isomalt-Sirup, wobei die Trockensubstanz des Sirups aus 7 bis 52 % (Gew./Gew.) 1,6-GPS, 24,5 bis 52 % (Gew./Gew.) 1,1-GPM, 0 bis 52 % (Gew./Gew.) 1,1-GPS, 0 bis 1,3 % (Gew./Gew.) Sorbit; 2,8 bis 13,8 % (Gew./Gew.) Maltit, 1,5 bis 4,2 % Gew./Gew.) Maltotriitol und 3,0 bis 13,5 % (Gew./Gew.) höheren Polyolen. Ein derartiger Sirup ist in der EP 1 194 042 B1 offenbart.

Bei dem erfindungsgemäßen zuckerfreien Kaugummi, der mit mindestens einer Schicht umhüllt ist, die ein schwer wasserlösliches Calciumsalz und/oder dessen Komposite umfasst, kann es sich beispielsweise um einen hartbeschichteten zuckerfreien Kaugummi handeln, der einen zuckerfreien Kaugummi-Kern und eine zuckerfreie Hartbeschichtung, die ein im Wesentlichen hygroskopisches zuckerfreies Süßungsmittel enthält, umfasst, wobei der Kaugummi-Kern einen Wassergehalt von weniger als etwa 2,5 Gew.-%, bezogen auf das Gewicht des Kerns, aufweist. Bei dem im Wesentlichen hygroskopischen Süßungsmittel kann es sich beispielsweise um Sorbit oder hydrierte Isomaltulose handeln. Derartige zuckerfreie hartbeschichtete Kaugummis sind in der WO 88/08671 beschrieben.

In einer weiteren Ausführungsform der Erfindung ist vorgesehen, dass sowohl die erfindungsgemäßen zuckerhaltigen Kaugummis als auch die erfindungsgemäßen zuckerfreien Kaugummis im Kaugummi selbst und/oder in der sie umhüllenden Schicht neben den vorstehend genannten Zucker-Arten und/oder Zuckeraustauschstoffen zusätzlich einen oder mehrere Intensivsüßstoffe enthalten können. Intensivsüßstoffe sind Verbindungen, die sich bei geringem beziehungsweise ver-nachlässigbar geringem Nährwert durch einen intensiven Süßgeschmack auszeichnen. Erfindungsgemäß ist insbesondere vorgesehen, dass der in den erfindungsgemäßen Kaugummis eingesetzte Intensivsüßstoff Cyclamat, beispielsweise Natriumcyclamat, Saccharin, zum Beispiel Saccharin-Natrium, Aspartame®, Glycyrrhizin, Neohesperidin-Dihydrochalcon, Thaumatin, Monellin, Acesulfam, Stevioside, Alitam, Sucralose oder ein Gemisch davon ist. Unter Verwendung solcher Intensivsüßstoffe kann insbesondere der Anteil an Zucker-Arten reduziert und trotzdem der vorwiegend süße Geschmack erhalten werden.

In einer weiteren Ausführungsform der Erfindung ist vorgesehen, dass der erfindungsgemäße Kaugummi nicht nur eine umhüllende Schicht, insbesondere dragierte Schicht, aufweist, die ein schwerlösliches Calciumsalz und/oder dessen Komposite umfasst, sondern mindestens 2 bis etwa 100 solcher umhüllender Schichten, insbesondere dragierter Schichten. Erfindungsgemäß ist es möglich, dass die einzelnen Schichten das gleiche Süßungsmittel beziehungsweise die gleichen Süßungsmittel aufweisen. Selbstverständlich besteht erfindungsgemäß auch die Möglichkeit, dass die einzelnen Schichten auch unterschiedliche Süßungsmittel enthalten können. Derart dragierte Kaugummi-Produkte werden also durch Schichtenfolgen unterschiedlicher Süßungsmittel-Zusammensetzung umhüllt. Durch eine geeignete Wahl der Reihenfolge und Anzahl der Beschichtungsschritte mit den unterschiedlichen Süßungsmitteln lassen sich Kaugummis mit gewünschten Eigenschaften gezielt herstellen.

Beispielsweise kann der erfindungsgemäße Kaugummi zunächst mit 1 bis etwa 45 dragierten Schichten umhüllt sein, die das 1,1-GPM-angereicherte Gemisch aus 1,6-GPS und 1,1-GPM enthalten. Anschließend werden auf diese Schichten 1 bis etwa 45 Schichten des 1,6-GPS-angereicherten Gemisches aus 1,6-GPS und 1,1-GPM aufgetragen. Ein derart dragierter Kaugummi zeichnet sich aufgrund der höheren Löslichkeit und größeren Süßkraft des die Außenschicht bildenden 1,6-GPS-angereicherten Gemisches durch eine insgesamt höhere Süßkraft im Vergleich zu beispielsweise mit hydrierter Isomaltulose beschichteten Kaugummis aus. Eine derartige Schichtenabfolge ist in der DE 195 32 396 C2 beschrieben.

Beispielsweise kann der erfindungsgemäße Kaugummi ein hartbeschichteter Kaugummi sein, wobei die Drageedecke mehrere Schichten, die etwa 50 % bis etwa 100 % Xylit enthalten, und mehrere Schichten, die etwa 50 % bis etwa 100 % hydrierte Isomaltulose enthalten, aufweist. Derartige Kaugummis sind in der WO 93/18663 offenbart.

In einer weiteren Ausführungsform ist vorgesehen, dass die einzelnen, den Kaugummi umhüllenden dragierten Schichten das gleiche Calciumsalz und/oder die gleichen Komposite davon enthalten. Erfindungsgemäß ist es selbstverständlich aber auch möglich, dass die einzelnen Schichten, die den Kaugummi umhüllen, unterschiedliche Calciumsalze und/oder unterschiedliche Komposite davon enthalten. Selbstverständlich besteht auch die Möglichkeit, dass einzelne Schichten kein Calciumsalz beziehungsweise keine Komposite davon enthalten.

Gemäß einer weiteren bevorzugten Ausführungsform enthält der Kaugummi neben dem schwer wasserlöslichen Calciumsalz und/oder dessen Kompositen zusätzlich mindestens ein Fluoridsalz. Überraschenderweise wurde gefunden, dass die Zugabe von Fluorid zu einer synergistischen Verstärkung des nukleierenden Effekts der schwerlöslichen Calciumsalze und/oder deren Kompositen führt. Insbesondere bevorzugt ist der Zusatz von Natrium- und/oder Kaliumfluorid. Bei gleichzeitiger Zugabe von schwer wasserlöslichem Calciumsalz und geringen Mengen Fluorid zeigt sich eine etwa fünffache synergistische Verstärkung. Bevorzugt sind erfindungsgemäß Mengen von 0,05 bis 0,15 Gew.-%, insbesondere von 0,08 bis 0,12 Gew.-% Fluoridsalz.

Gemäß einer weiteren bevorzugten Ausführungsform enthält der Kaugummi Aromastoffe, Füllstoffe und/oder weitere Hilfsstoffe (wie z.B. Glyzerin oder Mineralsalze, bspw. Zn²⁺ oder Mg²⁺).

Grundsätzlich können jegliche natürlichen oder naturidentischen Aromastoffe eingesetzt werden. Besonders bevorzugt können insbesondere Aromaöle wie z.B. Pfefferminzöl, Krauseminzöl, Eukalyptusöl, Anisöl, Fenchelöl, Kümmelöl und synthetische Aromaöle eingesetzt werden.

Auch Fruchtaromen können, insbesondere in festen oder flüssigen Fruchtzubereitungen, Fruchtextrakten oder Fruchtpulvern, enthalten sein. Bevorzugt sind dabei Ananas, Apfel, Aprikose, Banane, Brombeere, Erdbeere, Grapefruit, Heidelbeere, Himbeere, Maracuja, Orange, Sauerkirsch, rote und schwarze Johannisbeere, Waldmeister und Zitrone.

Es können auch Wirkstoffe, wie z.B. Menthol und/oder Vitamine, in dem erfindungsgemäßen Kaugummi enthalten sein. Ebenso können Organophosphonate, wie z.B. 1-Hydroxyethan-1,1-diphosphonsäure, Phosphonopropan-1,2,3-tricarbonsäure (Na-Salz) oder 1-Azacycloheptan-2,2-diphosphonsäure (Na-Salz), und/oder Pyrophosphate zugesetzt werden, die die Bildung von Zahnstein vermindern. Die erfindungsgemäßen Kaugummis können auch Acetylsalicylsäure als Wirkstoff enthalten.

Als Konservierungsstoffe können alle für Lebensmittel zugelassenen Konservierungsstoffe eingesetzt werden, bspw. Sorbin- oder Benzoesäure und deren Derivate, wie z.B. Natriumbenzoat und Parahydroxybenzoat (Natriumsalz), Schwefeldioxid oder schwefelige Säure, Natrium- oder Kaliumnitrit. Farbstoffe und Pigmente zur Erreichung eines ansprechenden Aussehens können ebenfalls enthalten sein.

Die vorliegende Erfindung betrifft ebenfalls ein Verfahren zur Herstellung von Kaugummis, die von mindestens einer Schicht, die mindestens ein schwer wasserlösliches Calciumsalz und/oder ein oder mehrer Komposite davon umfasst, wobei das schwer wasserlösliche Calciumsalz eine Teilchengröße kleiner 1000 nm aufweist umhüllt sind. Das erfindungsgemäße Verfahren zur Herstellung der erfindungsgemäßen Kaugummis umfasst das Herstellen eines Kaugummi-Kerns und das Umhüllen des Kaugummi-Kerns mit mindestens einer Schicht, die ein schwer wasserlösliches Calciumsalz und/oder ein Komposit davon umfasst.

Erfindungsgemäß ist vorgesehen, dass der Kaugummi-Kern nach herkömmlichen Verfahren hergestellt werden kann. Nach Herstellung der Kaugummi-Kerne werden die fertig hergestellten Kaugummi-Kerne vorzugsweise dragiert, wobei üblicherweise eingesetzte Dragierverfahren eingesetzt werden können. Beispielsweise können die fertig hergestellten Kaugümmi-Keme einer Weichdragierung, einer Hartdragierung oder einer Suspensionsdragierung unterzogen werden. Unter einer "Weichdragierung" wird das Aufbringen von in Wasser gelösten Sacchariden auf in Bewegung befindliche Kerne, insbesondere Kaugummi-Kerne, verstanden, wobei nach jedem Auftrag mit einem Saccharid-Pulver abgestreut wird, um die Feuchtigkeit zu binden. Durch diese Art der Dragierung entsteht eine weiche Dragee-Decke. Unter einer "Hartdragierung" wird ebenso wie bei der Weichdragierung das Aufbringen von in Wasser gelösten Sacchariden auf in Bewegung befindliche Kaugummi-Kerne verstanden, wobei jedoch kein Saccharid-Pulver aufgetragen wird, sondern unmittelbar die nicht-wässrigen Bestandteile angetrocknet werden. Wie bei der Weichdragierung wird eine Vielzahl verschiedener Einzelaufträge durchgeführt, zwischen denen mit Warm- oder Kaltluft getrocknet wird, so dass unterschiedlich dicke Dragee-Decken hergestellt werden können. Das Hartdragierungsverfahren kann auch mit zwei oder mehr unterschiedlichen Saccharid-Lösungen, die nacheinander aufgetragen werden, durchgeführt werden. Bei der "Suspensions-Dragierung" besteht die suspendierte Mischung aus einer flüssigen Phase, die zum Beispiel Zucker-Arten oder Zuckeraustauschstoffe gelöst in Wasser enthält, sowie einer festen Phase, die aus feinen kristallinen Teilen von Zucker-Arten und/oder Zuckeraustauschstoffen besteht. Charakteristisch für diese Art der Suspensions-Dragierung ist der getrennte Einsatz unterschiedlicher Saccharide.

In einer bevorzugten Ausführungsform des erfindungsgemäßen Verfahrens wird der Kaugummi-Kern mittels mindestens eines Hartdragier-Schrittes mit der das Calciumsalz und/oder dessen Komposit enthaltenden Schicht umhüllt. Der Hartdragier-Schritt umfasst das Aufbringen einer Lösung oder Suspension, die mindestens ein Süßungsmittel und das Calciumsalz und/oder Komposite davon enthält, und das anschließende Trocknen der aufgebrachten Lösung oder Suspension.

In einer weiteren Ausführungsform des erfindungsgemäßen Verfahrens wird der Kaugummi-Kern mittels mindestens eines Weichdragier-Schrittes mit der das Calciumsalz und/oder dessen Komposit enthaltenden Schicht umhüllt. Der Weichdragier-Schritt umfasst das Aufbringen einer Lösung oder Suspension, die mindestens ein Süßungsmittel enthält, und das Bestäuben der aufgebrachten Lösung oder Suspension mit einem Süßungsmittel-Pulver. In einer Ausgestaltung enthält die aufgebrachte Lösung oder Suspension die Gesamtmenge des Calciumsalzes und/oder von dessen Kompositen oder einen Teil davon. Das heißt, in dieser Ausgestaltung wird das Calcium-Salz und/oder dessen Komposit vollständig oder teilweise in die Lösung oder Suspension eingebracht und zusammen mit dieser auf die zu dragierenden Kaugummi-Kerne aufgetragen. In einer weiteren Ausgestaltung enthält das Süßungsmittel-Pulver die Gesamtmenge des Calciumsalzes und/oder von dessen Kompositen oder einen Teil davon. Das heißt, in dieser Ausgestaltung wird das Calcium-Salz und/oder dessen Komposit vollständig oder teilweise zusammen mit dem Süßungsmittel-Pulver zum Bestäuben der auf den Kaugummi-Kern aufgebrachten Lösung oder Suspension verwendet.

Erfindungsgemäß ist vorgesehen, dass die Hartdragier- oder Weichdragier-Schritte mehrmals wiederholt werden können, so dass die Kaugummi-Kerne mit jeweils mehreren umhüllenden Schichten versehen werden.

Das schwer wasserlösliche Calciumsalz ist erfindungsgemäß ausgewählt aus Fluorapatit, carbonathaltigem nichtstöchiometrischem Apatit, Hydroxylapatit und Fluor-dotierten Hydroxylapatit, wobei das Calciumsalz eine Teilchengröße kleiner 1000 nm, vorzugsweise von 5 bis 300 nm aufweist.

Die umhüllende Schicht enthält 0,001 bis 5 Gew.-%, vorzugsweise 0,01 bis 2 Gew.-% Calciumsalz und/oder eines Kompositen davon.

Der Kaugummi, der von einer Schicht umhüllt ist, die schwer wasserlösliches Calciumsalz und/oder dessen Komposite enthält, kann zur Zahnpflege und Zahnschonung sowie darüber hinaus zur Mineralisierung des Zahnschmelzes und/oder des Dentins verwendet werden. Einer kariösen Erkrankung der Zähne kann durch das Kauen des erfindungsgemäßen Kaugummis effektiv entgegengewirkt werden. Neben der Genussbefriedigung ist es möglich, den erfindungsgemäßen Kaugummi zusätzlich zur Kariesprophylaxe zu verwenden.

Folgende Beispiele sollen die Erfindung erläutern, ohne sie darauf zu beschränken:

### Beispiel 1

### 1. Herstellung eines Apatit-Protein-Komposits:

Zur Herstellung des Apatit-Gelatine-Komposits wird 2000 ml demineralisiertes Wasser in einem auf 25°C thermostatisierten 4 I Becherglas vorgelegt, in denen 44.10 g (0.30 Mol) CaCl₂·2H₂O (Fisher Chemicals p.a.) gelöst werden. Getrennt davon werden in 350 ml demineralisiertem Wasser 35 g Gelatine (Typ A, DGF-Stoess, Eberbach) bei etwa 50°C gelöst. Beide Lösungen werden vereinigt und stark mit einem Propellerrührer gerührt. Der pH-Wert wird mit verdünnter wässriger Base auf 7.0 eingestellt.

Zu dieser Gelatine- und Calciumsalzlösung werden unter starkem Rühren innerhalb von 120 min 300 mL einer 0.6 M (NH₄)₂HPO4-Lösung, die vorher auf pH 7.0 eingestellt wurde, mit einer automatisierten Zugabeapparatur gleichmäßig zugepumpt. Dabei wird der pH-Wert durch die geregelte Zugabe von verdünnter wässriger Base konstant auf pH 7.0 gehalten. Nach Beendigung der Zugabe wird weiter über 24 h gerührt.

Anschließend wird die Dispersion in Zentrifugenbecher gefüllt und der Feststoffanteil durch Zentrifugieren von der Lösung getrennt. Durch fünffaches Aufschütteln des Rückstandes in demineralisiertem Wasser und anschließendes erneutes Zentrifugieren werden die Salze weitgehend ausgewaschen, so dass kein Chlorid mehr nachweisbar ist.

### 2. Herstellung der Kaumasse:

Die Grundmasse wird auf 50°C vorerhitzt und der Doppel-Sigma-Schaufel-Kneter auf 40°C thermostatisiert. Die halbe Menge des Sorbits, das feste Mannit, die Grundmasse sowie das Lecithin werden miteinander 5 Minuten geknetet. Dann wird die Hälfte des Glycerins zugegeben. Nach weiteren 5 Minuten werden ein Viertel des Sorbits und die Hälfte des Glycerins zugefügt. Nach einer weiteren Minute Kneten werden ein Viertel von der Sorbitmenge sowie die andere Hälfte des Glycerins zugegeben. Diese Masse wird noch 5 Minuten geknetet, bevor der Maltitsirup zugegeben wird. Abschließend kommt nach weiteren 5 Minuten das Optamint® zur Masse. Diese wird noch weitere 5 Minuten verknetet.

Die Masse wird mit Talkum bestreut und noch im warmen Zustand auf 1.5 mm Dicke ausgerollt. Aus diesen Platten werden Streifen geschnitten. Die geschnittenen Platten wiegen etwa 2.5 bis 4.0 g und werden durch Pressen oder aber durch Rotieren in eine runde Form gebracht. Die so hergestellten Kaugummi-Kerne werden dann der Dragierung unterzogen.

**Tabelle 1: Kaumasse**

| Inhaltsstoffe | Menge |
|---|---|
| Kaugummi-Grundmasse Balear®-T (Cafosa, Spanien) | 33 g |
| D-Sorbit (98% rein, Aldrich) | 57.50 g |
| Mannit-Pulver (ABCR Chemikalien, Karlsruhe) | 5.00 g |
| Maltit-Sirup (85 Gew.-%) in Wasser (HDS-Chemie, Österreich) | 3.00 g |
| Glycerin, wasserfrei (Merck) | 2.00 g |
| Optamint® (Haarmann & Reimer GmbH) | 1.80 g |
| Lecithin (ACROS Organics) | 0.50 g |

Aus 250 g Saccharose und 40 mL Wasser wird bei 40°C eine gesättigte Lösung hergestellt (Rückstand wird abfiltriert). Zu der Lösung werden 2,5 g ApatitGelatine-Komposit-Partikel zugegeben. Der Dragierkessel mit 50 cm Durchmesser wird ebenfalls auf 40°C erhitzt und in Rotation versetzt. 500 g Kaugummis werden portionsweise zugegeben, wobei darauf zu achten ist, dass dieses Material nicht zu schnell eingeführt wird, um ein Verkleben zu verhindern. Die Zuckerlösung wird dann innerhalb von 5 h eingesprüht.

Eine entsprechende Dragierung wird auch mit gesättigten Lösungen der Zuckerersatzstoffe Maltit, Sorbit oder Isomalt bei sonst gleichen Bedingungen durchgeführt.

### Beispiel 2

### Herstellung von hart/weich-dragierten Kaugummis

Wie in Beispiel 1 beschrieben werden Kaugummi-Kerne hergestellt und dann einer Hart/Weich-Dragierung unterzogen. Dabei werden Dragees mit 33 Massen-%

Deckenanteil hergestellt.

### 1. Rezeptur für Dragierschicht

| **ROHSTOFFE** | **%** |
|---|---|
| ISOMALT ST-M | 0,60 |
| Wasser | 0,40 |
| Maltitsirup | 96,5 |
| Menthol-Aroma (Fa. Haamann-Reimer, Holzminden, deutschland | 0,5 |
| Apatit-Gelatine-Komposit gemäß Beispiel 1 | 1,00 |
| Titandioxid | 1,00 |
| ISOMALT ST-PF | zum Abstreuen |

### 2. Herstellung der Dragierlösung

Isomalt ST-M (Isomalt, bei dem 1,6-GPS und 1,1-GPM in im wesentlichen äquimolaren Mengen vorliegen und das eine mittlere Körnung aufweist, wobei der Durchmesser von ca. 90 % aller Partikel < 3 mm ist) wird unter Rühren in heißem Wasser aufgelöst und mit dem Maltitsirup gemischt. Anschließend werden Apatit-Gelatine-Komposit-Partikel, die wie in Beispiel 1 beschrieben hergestellt werden, Menthol-Aroma und TiO₂ in die Lösung gegeben. Die so erhaltene Lösung wird während des Dragierprozesses bei 30 bis 60°C gehalten, um eine bessere Verteilung der Lösung auf den Kaugummi-Kernen zu erreichen. Das Verhältnis der Isomalt-Lösung zu dem Maltit-Sirup kann je nach gewünschter Deckenhärte zwischen 100:0 und 1:99 variieren.

### 3. Dragierprozess

Im ersten Zyklus wird eine Auftragsmenge von 10-12 ml beziehungsweise 12,5-15,6 g Lösung/kg Kaugummi-Kerne aufgetragen, wobei die Kaugummi-Kerne vollständig befeuchtet werden. Danach wird die Lösung gründlich verteilt. Anschließend wird mit Isomalt ST-PF (5-8 g/kg Kaugummi-Kerne) bestäubt, bis die Oberfläche trocken ist. Isomalt ST-PF ist dadurch gekennzeichnet, dass 1,6-GPS und 1,1-GPM in im wesentlichen äquimolaren Mengen vorhanden sind, wobei eine pulverfeine Körnung vorliegt (Durchmesser von ca. 90 % aller Partikel < 100 µm).

Dieser Vorgang wird mit etwas verringerten Lösungs- und Pulvermengen in den nächsten drei Zyklen wiederholt. Danach werden weitere Zyklen durchgeführt werden, wobei steigende Lösungs- und Pulvermengen eingesetzt werden, bis das gewünschte Endgewicht nahezu erreicht ist. Anschließend wird die Oberfläche der dragierten Kaugummi-Kerne mit geringen Lösungsaufträgen ohne Pulverzugabe geglättet. Danach wird ein Polierprozess durchgeführt, wobei ca. 15 g Carnaubawachs zwischen den Dragees zerrieben werden. Um zu vermeiden, dass sich die Dragierdecke beim Polierprozess abschlägt, wird die Drehzahl der Trommel zu Beginn des Polierprozesses sehr niedrig eingestellt. Sobald das Poliermittel gut verteilt ist, wird die Rotationsgeschwindigkeit erhöht.

In einer Abwandlung des Verfahrens wird eine Lösung von Maltitsirup, Isomalt ST-M, Aromen und Farbstoffen hergestellt. Dieser Lösung wird dann ein Teil der vorgesehenen Menge der gemäß Beispiel 1 hergestellten Apatit-Gelatine-Komposit-Partikel zugegeben. Diese Lösung wird dann, wie vorstehend beschrieben, auf die Kaugummi-Kerne aufgetragen. Danach wird mit einem Pulvergemisch bestäubt, das Isomalt ST-PF und die restliche Menge der in die Dragierschicht einzuarbeitenden Apatit-Gelatine-Komposit-Partikeln enthält., In einer weiteren Abwandlung des Verfahrens wird zunächst eine Lösung hergestellt und auf die Kaugummi-Kerne aufgetragen, die Maltitsirup, Isomalt ST-M, Aromen und Farbstoffe enthält, nicht jedoch die Apatit-Gelatine-Komposit-Partikel. Nach dem Auftragen dieser Lösung auf die Kaugummi-Keme wird dann entweder mit einem Pulvergemisch aus Isomalt ST-PF und den in die Dragierschicht einzuarbeitenden Apatit-Gelatine-Komposit-Partikeln oder nur den Apatit-Gelatine-Komposit-Partikein (als sogenannte "Dry Charge") in trockener Form bestäubt.

### Beispiel 3

### Weichdragierung von Kaugummi-Kernen

Die in Beispiel 1 hergestellten Kaugummi-Kerne werden in diesem Beispiel einer Weichdragierung unterzogen. Dabei werden Dragees mit 33 Massen-% Deckenanteil hergestellt.

### 1. Rezeptur für Dragierschicht

| **ROHSTOFFE** | **%** |
|---|---|
| Maltitsirup | 97,5 |
| Menthol-Aroma (Fa. Haamann-Reimer, Holzminden, Deutschland) | 0,5 |
| Titandioxid | 1 |
| ISOMALT ST-PF | zum Abstreuen |
| Apatit-Gelatine-Komposit gemäß Beispiel 1 | 1 |

### 2. Vorbehandlung von Maltitsirup

Damit sich der Maltitsirup besser auf den zu dragierenden Kaugummi-Kernen verteilt, wird er vor dem Dragieren auf Temperaturen von etwa 40°C bis 50°C erwärmt.

### 3. Dragierprozess

Der Dragierprozess wird durchgeführt wie in Beispiel 2 beschrieben.

Auch hier kann das Verfahren abgewandelt werden, indem ein Teil der in die Dragierschicht einzuarbeitenden Apatit-Gelatine-Komposit-Partikel dem Maltit-Sirup zugemischt wird und die restliche Menge der Apatit-Gelatine-Komposit-Partikel in trockener Form entweder allein oder zusammen mit dem lsomalt ST-PF-Pulver zum Bestäuben eingesetzt wird. In einer weiteren Abwandlung des Verfahrens wird der Maltit-Sirup ohne die Apatit-Gelatine-Komposit-Partikel auf die Kaugummi-Keme aufgetragen. Die in die Dragierschicht einzuarbeitende Gesamtmenge der Apatit-Gelatine-Komposit-Partikel wird dann in trockner Form zum Bestäuben eingesetzt.

### 4. Vorgummierung

Bei der Weichdragierung kann eine Vorgummierung vorgenommen werden. Die Vorgummierung kann wie folgt durchgeführt werden:
1) auf die nicht-dragierten Kaugummi-Kerne werden zunächst eine Gummi arabicum-Lösung (40%-ig) und eine Isomalt-Lösung (60%-ig) im Verhältnis 1:1 aufgetragen und dann wird mit Isomalt ST-PF abgestreut oder
2) es wird eine Gummi arabicum-Lösung (25-40%-ig) auf die nicht-dragierten Kaugummi-Kerne aufgetragen und dann wird mit Isomalt ST-PF abgestreut oder
3) es wird eine Gummi arabicum-Lösung (25-40 %-ig) aufgetragen und dann nur mit Gummi arabicum abgestreut.

Normalerweise werden 1 bis 4 Vorgummier-Zyklen durchgeführt. Vor dem Dragieren werden die vorgummierten Kaugummi-Kerne gut abgetrocknet, wobei vorzugsweise eine Zwischenlagerung über Nacht in trockener Umgebung erfolgt.

### Beispiel 4

### Dragierung mit einer Isomalt GS-Lösung

Es werden Dragees mit 33 Massen-% Deckenanteil hergestellt.

### 1. Rezeptur für Dragierschicht

| **ROHSTOFFE** | **%** |
|---|---|
| ISOMALT GS | 65,00 |
| Apatit-Gelatine-Komposit gemäß Beispiel 1 | 1,00 |
| Wasser | 28,80 |
| Aspartam | 0,05 |
| Acesulfam K | 0,05 |
| Gummi arabicum 50 % | 4,10 |
| TiO₂ | 1,00 |
| GESAMT | 100,00 |

In diesem Beispiel wurde ebenso wie in den nachfolgenden Beispielen 5, 6 und 7 auf den Zusatz von Aromastoffen verzichtet.

### 2. Herstellung der Lösung

Zunächst werden Isomalt GS (1,6-GPS-angereichertes Gemisch aus 1,1-GPM und 1,6-GPS mit einem 1,6-GPS-Gehalt > 57%), Süßstoffe und Wasser gerührt und erhitzt, bis eine kristallfreie Lösung vorliegt (70-80°C). Danach wird die Energiezufuhr eingestellt. Dann werden eine Gummi arabicum-Lösung, TiO₂ sowie die in Beispiel 1 hergestellten Apatit-Gelatine-Komposit-Partikel zugegeben und so lange gerührt, bis das Gemisch homogen ist. Die Endtemperatur der Lösung beträgt etwa 50°C. Während der gesamten Dragierdauer wird die Lösung zirkuliert oder gerührt und bei etwa 45 bis 55°C gehalten. Bei Verwendung automatischer Systeme müssen dementsprechend die Rohrleitungen beheizbar sein.

### 3. Dragierprozess

Da bestimmte Einlagen während der ersten vier Dragierzyklen zu Klebrigkeit und aufgrund dessen zur Bildung von Agglomeraten tendieren, nachdem die Lösung zugegeben und verteilt wurde, erfolgt nach ausreichender Verteilung der hergestellten Lösung auf der Oberfläche der Kaugummi-Kerne eine Bestäubung mit Isomalt ST-PF, gegebenenfalls in Kombination mit Apatit-Gelatine-Komposit-Partikeln.

Im ersten Dragierzyklus wird eine Menge von 10-12 ml beziehungsweise 12,5-15,6 g Lösung/kg Kaugummi-Kerne aufgetragen, damit die Einlagen vollständig feucht sind. Danach wird die Lösung gründlich verteilt. Zur Vermeidung von Agglomerat-Bildung werden die Kaugummi-Kerne mit gesiebtem Isomalt ST-PF (10-15 g/kg Kaugummi-Kerne) bestäubt. Nach ausreichender Verteilung wird getrocknet.

In den nachfolgenden drei Zyklen wird die Lösungsmenge auf 7-10 ml verringert und die Pulvermenge auf 9-13 g/kg Kaugummi-Kerne. Während des weiteren Dragierprozesses wird die Lösungsmenge/Phase erhöht, bis das gewünschte Gewicht der Dragierschicht nahezu erreicht ist. In den letzten zwei bis drei Phasen werden die dragierten Kaugummis durch Reduktion der Lösungsmenge und längere Verteilpausen geglättet. Zur Erzielung einer guten Knusprigkeit und eines dauerhaften Glanzes wird ein Jogging-Schritt bei etwa 3 x 4 UpM mit Trockenluft über einen Zeitraum von 15 bis 30 Minuten durchgeführt. Der Staub, der durch die Intensivtrocknung entstanden ist, wird mit einem Lösungsauftrag gebunden. Danach wird ein Trocknungsschritt ohne Zuführung von Trockenluft durchgeführt, bis die Oberfläche trocken und staubfrei ist. Danach wird ein Polierprozess durchgeführt, wobei ca. 15 g Carnaubawachs zwischen den Dragees zerrieben werden. Um zu vermeiden, dass sich die Dragierdecke beim Polierprozess abschlägt, wird die Drehzahl der Trommel zu Beginn des Polierprozesses sehr niedrig eingestellt. Sobald das Poliermittel gut verteilt ist, wird die Rotationsgeschwindigkeit erhöht.

In einer Abwandlung des Verfahrens wird eine Lösung von Isomalt GS, Süßstoffen, Gummi arabicum-Lösung,TiO₂, Aromen und Farbstoffen hergestellt. Dieser Lösung wird dann ein Teil der vorgesehenen Menge der Apatit-Gelatine-Komposit-Partikel zugegeben. Diese Lösung wird dann, wie vorstehend beschrieben, auf die Kaugummi-Kerne aufgetragen. Danach wird mit einem Pulvergemisch bestäubt, das Isomalt ST-PF und die restliche Menge der in die Dragierschicht einzuarbeitenden Apatit-Gelatine-Komposit-Partikeln enthält. In einer weiteren Abwandlung des Verfahrens wird zunächst eine Lösung hergestellt und auf die Kaugummi-Kerne aufgetragen, die Isomalt GS, Süßstoffen, Gummi arabicum-Lösung,TiO₂, Aromen und Farbstoffe enthält, nicht jedoch die Apatit-Gelatine-Komposit-Partikel. Nach dem Auftragen dieser Lösung auf die Kaugummi-Keme wird dann entweder mit einem Pulvergemisch aus Isomalt ST-PF und den in die Dragierschicht einzuarbeitenden Apatit-Gelatine-Komposit-Partikeln oder nur den Apatit-Gelatine-Komposit-Partikeln (als sogenannte "Dry Charge") in trockener Form bestäubt.

Das Verfahren kann sowohl für herkömmliches offenes Dragieren als auch für geschlossene Dragiersysteme eingesetzt werden.

Auch bei diesem Verfahren kann ein Vorgummierungs-Schritt durchgeführt werden, wie in Beispiel 3 beschrieben.

### Beispiel 5

### Dragierung von Kaugummi-Kernen mit einer Isomalt GS-Suspension

Es werden Dragees mit 33 Massen-% Deckenanteil hergestellt.

### 1. Rezeptur für Dragierschicht

| **ROHSTOFFE** | **%** |
|---|---|
| ISOMALT GS | 43,65 |
| Apatit-Gelatine-Komposit gemäß Beispiel 1 | 1,00 |
| Wasser | 28,00 |
| Aspartam | 0,05 |
| Acesulfam K | 0,05 |
| Gummi arabicum 50 % | 4,10 |
| ISOMALT ST-PF | 22,15 |
| TiO₂ | 1,00 |
| Gesamt | 100,00 |

### 2. Herstellung der Suspension

Isomalt GS, Süßstoffe und Wasser werden gerührt und erhitzt, bis eine kristallfreie Lösung vorliegt (70-80°C). Danach wird die Energiezufuhr eingestellt. Anschließend werden eine Gummi arabicum-Lösung, Apatit-Gelatine-Komposit-Partikel, Isomalt ST-PF und TiO₂ zugegeben und gerührt, bis eine homogene Masse vorliegt. Die Endtemperatur der Suspension beträgt etwa 40°C. Während der gesamten Dragierdauer wird die Suspension zirkuliert oder gerührt und bei etwa 40 bis 45°C gehalten.

### 3. Dragierprozess

Im ersten Zyklus wird eine Auftragsmenge von 10-12 ml beziehungsweise 12,5-15,6 g Suspension/kg Kaugummi-Kerne aufgetragen, damit die Einlagen vollständig befeuchtet werden. Dann wird die Suspension gründlich verteilt. Zur Vermeidung von Agglomeratbildung werden die Kaugummi-Kerne mit gesiebtem Isomalt ST-PF (10-15 g/kg Kaugummi-Kerne) bestäubt. Anschließend erfolgt eine Trocknung. In den nachfolgenden drei Zyklen wird die Suspensionsmenge auf 7-10 ml und die Pulvermenge auf 9-13 g/kg Kaugummi-Kerne verringert. Während des weiteren Dragierprozesses wird die Suspensionsmenge/Phasen erhöht, bis das gewünschte Gewicht der Drageedecke erreicht ist. In den letzten zwei bis drei Phasen werden die dragierten Kaugummis durch Reduktion der Suspensionsmenge und längere Verteilpausen geglättet. Eine gute Knusprigkeit und ein dauerhafter Glanz werden mittels eines Jogging-Schrittes bei etwa 3 x 4 UpM mit Trockenluft über einen Zeitraum von 15 bis 30 Minuten erzielt. Der Staub, der durch die intensive Trocknung entstanden ist, wird mit einem letzten Suspensionsauftrag gebunden. Danach wird ohne Zuführung von Trockenluft getrocknet, bis die Oberfläche trocken und staubfrei ist. Danach wird ein Polierprozess durchgeführt, wobei ca. 15 g Carnaubawachs zwischen den Dragees zerrieben werden.

Auch hier kann das Verfahren abgewandelt werden, indem nur ein Teil der vorgesehenen Gesamtmenge der Apatit-Gelatine-Komposit-Partikel in die Suspension gegeben wird und die restliche Menge der Apatit-Gelatine-Komposit-Partikel zum Bestäuben eingesetzt wird.

Selbstverständlich kann die Gesamtmenge der in die Dragierschicht einzuarbeitenden Apatit-Gelatine-Komposit-Partikel auch vollständig zum Bestäuben eingesetzt werden.

Auch hier kann eine Vorgummierung erfolgen, wie in Beispiel 3 beschrieben.

### Beispiel 6

### Dragierung von Kaugummi-Kernen mit Isomalt ST-Lösung

Es werden Dragees mit 33 Massen-% Deckenanteil hergestellt.

### 1. Rezeptur für Dragierschicht

| **ROHSTOFFE** | **%** |
|---|---|
| ISOMALT ST-M | 65,00 |
| Apatit-Gelatine-Komposit gemäß Beispiel 1 | 1,00 |
| Wasser | 28,80 |
| Aspartam | 0,05 |
| Acesulfam K | 0,05 |
| Gummi arabicum 50 % | 4,10 |
| TiO₂ | 1,00 |
| GESAMT | 100,00 |

### 2. Herstellung der Lösung

Isomalt ST-M, Süßstoffe und Wasser werden gerührt und erhitzt, bis eine kristallfreie Lösung vorliegt (70-80°C). Danach wird die Energiezufuhr eingestellt. Eine Gummi arabicum-Lösung, Apatit-Gelatine-Komposit-Partikel und TiO₂ werden zugegeben und gerührt, bis eine homogene Masse vorliegt. Die Endtemperatur der Lösung beträgt etwa 60°C. Die Lösung wird während der gesamten Dragierdauer zirkuliert oder gerührt und bei etwa 60 bis 70°C gehalten.

### 3. Dragierprozess

Im ersten Zyklus wird eine Auftragsmenge von 10-12 ml beziehungsweise 12,5-15,6 g Lösung/kg Kaugummi-Kerne aufgetragen, wobei die Einlagen vollständig befeuchtet werden. Danach wird die Lösung gründlich verteilt. Zur Vermeidung von Agglomeratbildung werden die Einlagen mit gesiebtem Isomalt ST-PF (10-15 g/kg Kaugummi-Kerne) bestäubt. Anschließend erfolgt eine Verteilung und Trocknung.

In den nächsten drei Zyklen wird die Lösungsmenge auf 7-10 ml und die Pulvermenge auf 9-13 g/kg Kaugummi-Kerne verringert. Während es weiteren Dragierprozesses wird die Lösungsmenge/Phase erhöht, bis das gewünschte Gewicht der Drageedecke nahezu erreicht ist. In den letzten zwei bis drei Phasen werden die dragierten Kaugummis durch Reduktion der Lösungsmenge und längere Verteilpausen geglättet. Durch die Durchführung eines Jogging-Schrittes bei etwa 3 x 4 UpM mit Trockenluft über einen Zeitraum von 15 bis 30 Minuten wird eine gute Knusprigkeit und ein dauerhafter Glanz erzielt. Der Staub, der durch die Intensivtrocknung entstanden ist, wird mit einem Lösungsauftrag gebunden. Danach wird ohne Zuführung von Trockenluft getrocknet, bis die Oberfläche trocken und staubfrei ist. Danach wird ein Polierprozess durchgeführt, wobei ca. 15 g Carnaubawachs zwischen den Dragees zerrieben werden. Um zu vermeiden, dass sich die Decke während des Polierprozesses abschlägt, wird die Drehzahl der Trommel zu Beginn des Polierprozesses relativ niedrig eingestellt. Sobald das Poliermittel gut verteilt ist, kann die Rotationsgeschwindigkeit erhöht werden.

Das Verfahren kann abgewandelt werden, indem nur ein Teil der vorgesehenen Gesamtmenge der Apatit-Gelatine-Komposit-Partikel in die Lösung gegeben wird und die restliche Menge der Apatit-Gelatine-Komposit-Partikel zum Bestäuben eingesetzt wird. In einer weiteren Abwandlung kann die Gesamtmenge der in die Dragierschicht einzuarbeitenden Apatit-Gelatine-Komposit-Partikel vollständig zum Bestäuben eingesetzt werden.

Auch hier kann eine Vorgummierung wie in Beispiel 3 beschrieben durchgeführt werden.

### Beispiel 7

### Dragierung von Kaugummi-Kernen mit Isomalt ST-Suspension

Es werden Dragees mit 33 Massen-% Deckenanteil hergestellt.

### 1. Rezeptur für Dragierschicht

| **ROHSTOFFE** | **%** |
|---|---|
| ISOMALT ST-M | 43,65 |
| Apatit-Gelatine-Komposit gemäß Beispiel 1 | 1,00 |
| Wasser | 28,00 |
| Aspartam | 0,05 |
| Acesulfam K | 0,05 |
| *Gummi arabicum* 50 % | 4,10 |
| TiO₂ | 1,00 |
| GESAMT | 100,00 |

### 2. Herstellung der Suspension

Isomalt ST-M, Süßstoffe und Wasser werden gerührt und erhitzt, bis eine kristallfreie Lösung (70-80°C) vorliegt. Danach wird die Energiezufuhr eingestellt. Die Gummi arabicum-Lösung, Isomalt ST-PF, die gemäß Beispiel 1 hergestellten Apatit-Gelatine-Komposit-Partikel und TiO₂ werden zugegeben und gerührt, bis eine homogene Masse vorliegt. Die Endtemperatur der Suspension beträgt etwa 55°C. Bei einer gesamten Dragierdauer wird die Suspension zirkuliert oder gerührt und bei etwa 50-60°C gehalten.

### 3. Dragierprozess

Im ersten Zyklus wird eine Auftragsmenge von 10-12 ml beziehungsweise 12,5-15,6 g Suspension/kg Kaugummi-Kernen aufgetragen, wobei die Einlagen vollständig befeuchtet werden. Danach wird die Suspension gründlich verteilt. Zur Vermeidung von Agglomeratbildung werden die Einlagen mit gesiebtem Isomalt ST-PF (10-15 g/kg Kaugummi-Kerne) bestäubt. Anschließend wird verteilt und getrocknet. In den nächsten drei Zyklen wird die Suspensionsmerige auf 7 bis 10 ml und die Pulvermenge auf 9-13 g/kg Kaugummi-Kerne verringert. Während des weiteren Dragierprozesses wird die Suspensionsmenge/Phase erhöht, bis das gewünschte Gewicht der Drageedecke fast vollständig erreicht ist. In den letzten 2-3 Phasen werden die dragierten Kaugummis durch Reduktion der Suspensionsmenge und längere Verteilpausen geglättet. Anschließend wird ein Jogging-Schritt bei ca. 3 x 4 UpM mit Trockenluft über einen Zeitraum von 15 bis 30 Minuten durchgeführt. Der Staub, der durch die intensive Trocknung entstanden ist, wird mit einem letzten Suspensionsauftrag gebunden. Danach wird ohne Zuführung von Trockenluft getrocknet, bis die Oberfläche trocken und staubfrei ist. Danach wird ein Polierprozess durchgeführt, wobei ca. 15 g Carnaubawachs zwischen den Dragees zerrieben werden.

Das Verfahren kann abgewandelt werden, indem nur ein Teil der vorgesehenen Gesamtmenge der Apatit-Gelatine-Komposit-Partikel in die Suspension gegeben wird und die restliche Menge der Apatit-Gelatine-Komposit-Partikel zum Bestäuben eingesetzt wird. In einer weiteren Abwandlung kann die Gesamtmenge der in die Dragierschicht einzuarbeitenden Apatit-Gelatine-Komposit-Partikel vollständig zum Bestäuben eingesetzt werden.

Auch hier kann ein Vorgummierungs-Schritt, wie in Beispiel 3 beschrieben, durchgeführt werden.

### Beispiel 8

### Herstellung von Kaugummi-Dragees mittels einer halbautomatischen Dragieranlage

Es wurden Kaugummi-Einlagen der Fa. Gum Base, Italien, mit einer Drageedecke, die 1 % Apatit-Gelatine-Komposit-Partikel enthielt, versehen.

Tabelle 2 zeigt eine Übersicht der durchgeführten Dragierungen mit unterschiedlichen Süßungsmitteln.

**Tabelle 2: Übersicht der Dragierungen mit unterschiedlichen Zuckern beziehungsweise Zuckeralkoholen**

| **Rohstoff** | **Medium¹** | **T_{A} [°C]** | **TS [%]** |
|---|---|---|---|
| Saccharose | Lösung | 60 | 72 |
| Maltit | Lösung³ | 60 | 71 |
| Maltit | Lösung³ | 60 | 72 |
| Maltit | Lösung³ | 60 | 71 |
| Maltit | Lösung³ | 60 | 69 |
| Maltit | Lösung³ | 60 | 71 |
| Maltit | Suspension² | 45 | 74 |
| Isomalt ST | Lösung⁴ | 70 | 67 |
| Isomalt ST | Suspension² | 65 | 67 |
| Isomalt GS | Lösung⁴ | 50 | 67 |
| Isomalt GS | Suspension² | 40 | 67 |
| Isomalt GS | Lösung⁴ | 65 | 72 |
| Isomalt GS | Lösung⁴ | 55 | 67 |
| Isomalt GS | Lösung⁴ | 55 | 67 |
| Isomalt GS | Suspension² | 47 | 72 |

| | | | |
|---|---|---|---|
| ¹: alle Medien enthalten 2 % Gummi arabicum und 1 % Apatit-Gelatine-Komposit ²: Verhältnis gelöstes Polyol zu Feststoff beträgt 2:1 ³: enthält 0,5 % Titandioxid ⁴: enthält 1 % Titandioxid | | | |

### Präparation der Dragiermedien

Die Dragiermedien wurden in einem Edelstahlkochtopf (V = 14 I) zubereitet. Die Zubereitung wird für die reproduzierbare Herstellung von Dragiermedien schematisiert, aber separat definiert für Lösung und Suspension, durchgeführt. Als erstes wird die benötigte Menge VE-Wasser erhitzt (70°C bei Isomalt GS-Rezepturen und 80°C bei Isomalt ST-Rezepturen). Die zu lösende Menge Zuckeralkohol, vermischt mit eventuell verwendeten festen Zusätzen, z.B. Gummi arabicum, wird hinzugefügt. Bei der Herstellung einer Lösung wird die gesamte Polyolmenge portionsweise eingestreut und gelöst. Unter permanenter Agitation mit einem Propellerrührer bei maximal 250 min⁻¹, angetrieben mit einem Rührwerk Eurostar der Fa. IKA, kühlt die Mischung bis auf 5-7 K > Arbeitstemperatur (T_{A}) ab. Bei der Herstellung einer Suspension wird das Pulver (disperse Phase) portionsweise hinzugefügt. Vor dieser Dosage wird die Heizplatte ausgeschaltet, damit nicht zu viel vom eingestreuten Feststoff ebenfalls in Lösung geht. Die Abkühlung des Systems auf die Arbeitstemperatur wird durch die Lösungsenthalpie des Feststoffs unterstützt. Die Temperatur wird mit einem Einstech-Pt 100 der Fa. TESTOTHERM kontrolliert. Die Suspension hat eine gelblich-milchige Farbe, während die Lösung klargelblich ist. Zum Schluss werden der Farbstoff und die Apatit-Gelatine-Komposit-Partikel zugegeben. Nach deren vollständiger Dispergierung wird das Dragiermedium in den vorgeheizten Vorlagebehälter des Coaters über ein Sieb überführt. Anschließend folgt die Kalibrierung der Dosiereinrichtung des Coaters.

### Verwendete Dragieranlage

Zur Dragierung wird ein DRIACOATER Vario 500/600 der Fa. DRIAM eingesetzt. Dabei handelt es sich um eine halbautomatische Dragieranlage, bestehend aus einer Rechneranlage, einem Temperier-, Dosier- und Luftführungssystem und einer perforierten, nonagonalen Trommel. Zur Peripherie gehört eine Einheit zur Konditionierung der Trocknungsluft. Das Arbeitsvolumen des DRC Vario 500/600 beträgt 10 kg für Kaugummi-Kerne.

Eine Membran-Dosierpumpe fördert das Dragiermedium aus dem Vorlagebehälter LT bi 10 zu den zwei pneumatisch betriebenen Einstoffdüsen der Fa. SPRAYING SYSTEM Typ 1/8 JJ AUH-SS. Die Stabilität und Höhe des Förderdruckes und somit die resultierende Sprühfläche ist von der Viskosität des Dragiermediums und dem verwendeten Düsenmundstück abhängig. Vorrangig wird das Mundstück Typ TEEJET 9501, d = 0,66 mm, verwendet. Bei zu niedrigen Drücken wird das Mundstück 800067 mit einem Durchmesser von 0,53 mm eingesetzt. Der gesamte Sprüharm, seine Versorgungsleitungen und der Vorlagebehälter sind durch ein Doppelmantelsystem kontrolliert temperiert. Das Dragiermedium wird im Vorlagebehälter unter ständigem Rühren in Bewegung gehalten.

Das vorhandene Luftführungssystem des Coaters ermöglicht zwei Varianten der Trocknung des Guts: eine Gleichstromtrocknung, bei der die Luft die gleiche Richtung wie der Sprühstrahl besitzt, oder eine Gegenstromtrocknung, bei der die Luft durch die perforierte Trommelwand durch das Gut hindurch strömt. Alle Dragierungen wurden in diesem Beispiel unter Verwendung der Gleichstromtrocknung durchgeführt. Die zur Trocknung eingesetzte Luft wird zuvor konditioniert, wobei die Luft in der Temperiereinheit zunächst auf die geforderte Temperatur gekühlt oder geheizt wird. Danach wird die gewünschte Wassermenge mittels. Dampf in die temperierte Luft eingespeist. Die so behandelte Luft kann dann als konditionierte Luft (gewünschter Taupunkt) zur Trocknung eingesetzt werden.

Über den Leitrechner wird mit Hilfe der Software OCTOPUS 3000 die Programmierung und Speicherung von Dragierprogrammen gewährleistet. Außerdem werden kontinuierlich bestimmte Messwerte, beispielsweise Ablufttemperatur und Abluftfeuchte, aufgezeichnet.

### Dragierung

Zunächst wird, wie vorstehend beschrieben, das Dragiermedium hergestellt. In der Zwischenzeit werden die Kaugummi-Kerne in der rotierenden und durchlüfteten Trommel vom Talkum befreit und geglättet. Nach Überführung des Dragiermediums in den Vorlagebehälter wird das Dosiersystem kalibriert.

Tabelle 3 zeigt die zur Dragierung eingesetzten Parameter.

**Tabelle 3: Arbeitsbedingungen bei Dragierungen mit DRC Vario 500/600**

| **Parameter** | **Einstellung** |
|---|---|
| Trommelgeschwindigkeit [min⁻¹] | 20 |
| Volumenstrom der Trocknungsluft [m³/min] | 5 |
| Taupunkt [°C] | -2 bis 0 |
| Temperatur der Trocknungsluft [°C] | 25 |
| Differenzdruck in der Trommel [mbar] | 4 |
| Chargengröße (Kerne) [kg] | 8 |

Während der ersten drei Zyklen werden nach der Hälfte der Zeit in der Verteilungsphase ca. 70 g Pulver des verwendeten Zuckeraustauschstoffes über die feuchten Kerne gestreut, da die Klebeneigung zu diesem Zeitpunkt sehr stark ist. Außerdem können während dieser Zyklen anhaftende Kerne von der Trommel-Innenwand entfernt werden.

Es werden Dragees mit 33 Massen-% Deckenanteil hergestellt. Alle Dragierungen werden mit einer Polierphase beendet, wobei ca. 15 g Carnaubawachs zwischen den Dragees zerrieben werden.

## Patentansprüche

1. Kaugummi, **dadurch gekennzeichnet, dass** er von mindestens einer Schicht umhüllt ist, wobei diese Schicht schwer wasserlösliches Calciumsalz und/oder dessen Komposite umfasst, wobei das schwer wasserlösliche Calciumsalz eine Teilchengröße kleiner 1000 nm aufweist.

2. Kaugummi nach Anspruch 1, **dadurch gekennzeichnet, dass** das Calciumsalz ausgewählt ist aus Fluorapatit, carbonathaltigem nichtstöchiometrischem Apatit, Hydroxylapatit und Fluor-dotiertem Hydroxylapatit.

3. Kaugummi nach Anspruch 2, **dadurch gekennzeichnet, dass** das schwer wasserlösliche Calciumsalz eine Teilchengröße von 5 bis 300 nm aufweist.

4. Kaugummi nach einem der vorstehenden Ansprüche, **dadurch gekennzeichnet, dass** das schwer wasserlösliche Calciumsalz in Form von stäbchenförmigen Kristallen vorliegt.

5. Kaugummi nach einem der vorstehenden Ansprüche, **dadurch gekennzeichnet, dass** die umhüllende Schicht 0,001 bis 5 Gew.-%, insbesondere 0,01 bis 2 Gew.-% Calciumsalz und/oder dessen Komposite enthält.

6. Kaugummi nach einem der vorstehenden Ansprüche, **dadurch gekennzeichnet, dass** die umhüllende Schicht ein Komposit aus Calciumsalz und Proteinkomponente enthält.

7. Kaugummi nach Anspruch 6, **dadurch gekennzeichnet, dass** die Proteinkomponente ausgewählt ist aus Gelatine, Casein oder deren Hydrolysaten, insbesondere Gelatine.

8. Kaugummi nach einem der vorhergehenden Ansprüche, **dadurch gekennzeichnet, dass** das Calciumsalz und/oder dessen Komposite von einem oder mehreren Oberflächenmodifikationsmitteln umhüllt sind.

9. Kaugummi nach einem der vorstehenden Ansprüche, **dadurch gekennzeichnet, dass** die mindestens umhüllende Schicht eine dragierte Schicht ist.

10. Kaugummi nach einem der Ansprüche 1 bis 9, wobei der Kaugummi zuckerhaltig ist.

11. Kaugummi nach Anspruch 10, wobei der Kaugummi und/oder die umhüllende Schicht als Süßungsmittel Saccharose, Invertflüssigzucker, Invertzuckersirup, Glucose, Glucose-Sirup, Polydextrose, Tagatose, Trehalose, Trehalulose, Maltose, Lactose, Fructose, Leucrose, Palatinose, kondensierte Palatinose, hydrierte kondensierte Palatinose, oder ein Gemisch davon enthalten.

12. Kaugummi nach Anspruch 10 oder 11, wobei der Kaugummi und/oder die umhüllende Schicht als zusätzliches Süßungsmittel Fructooligosaccharide, Lactit, Sorbit, Xylit, Mannit, Maltit, Erythrit, 6-O-α-D-Glucopyranosyl-D-sorbit (1,6-GPS), 1-O-α-D-Glucopyranosyl-D-sorbit (1,1-GPS), 1-O-α-D-Glycopyranosyl-D-mannit (1,1-GPM) oder ein Gemisch davon enthalten.

13. Kaugummi nach einem der Ansprüche 1 bis 9, wobei der Kaugummi zuckerfrei ist.

14. Kaugummi nach Anspruch 13, wobei der Kaugummi und die umhüllende Schicht als Süßungsmittel Fructooligosaccharide, Lactit, Sorbit, Xylit. Mannit, Maltit, Erythrit, 6-O-α-D-Glucopyranosyl-D-sorbit (1,6-GPS), 1-O-α-D-Glucopyranosyl-D-sorbit (1,1-GPS), 1-O-α-D-Glycopyranosyl-D-mannit (1,1-GPM) oder ein Gemisch davon enthalten,

15. Kaugummi nach Anspruch 12 oder 14, wobei die Gemische ausgewählt sind aus der Gruppe bestehend aus einem äquimolaren oder nahezu äquimolaren Gemisch von 1,6-GPS und 1,1-GPM (Isomalt), einem Gemisch aus 1,6-GPS, 1,1-GPS und 1,1-GPM, einem 1,6-GPS-angereichertem Gemisch aus 1,6-GPS und 1,1-GPM mit einem 1,6-GPS-Anteil von 57 Gew.-% bis 99 Gew.-% und einem 1,1-GPM-Anteil von 43 Gew.-% bis 1 Gew.-%, einem 1,1-GPMangereichertem Gemisch aus 1,6-GPS und 1,1-GPM mit einem 1,6-GPS-Anteil von 1 Gew.-% bis 43 Gew.-% und einem 1,1-GPM-Anteil von 57 Gew.-% bis 99 Gew.-%, und einem Sirup, bestehend aus einem Gemisch aus hydriertem Stärkehydrolysatsirup und Isomalt-Sirup oder Isomalt-Pulver, wobei die Trockensubstanz des Sirups aus 7-52% (Gew./Gew.) 1,6-GPS, 24,5-52 % (Gew./Gew.) 1,1-GPM, 0-52 % (Gew./Gew.) 1,1-GPS, 0-13 % (Gew./Gew.) Sorbit, 2,8-13,8 % (Gew./Gew.) Maltit, 1,5-4,2 % (Gew./Gew.) Maltotriitol und 3,0-13,5 % (Gew./Gew.) höheren Polyolen besteht.

16. Kaugummi nach einem der vorstehenden Ansprüche, wobei der Kaugummi und/oder die umhüllende Schicht zusätzlich einen oder mehrere Intensivsüßstoffe enthalten.

17. Kaugummi nach Anspruch 16, wobei der Intensivsüßstoff Cyclamat, Saccharin, Aspartam, Glycyrrhizin, Neohesperidin-Dihydrochaicon, Steviosid, Thaumatin, Monellin, Acesulfam, Alitam, Sucralose oder ein Gemisch davon ist.

18. Kaugummi nach einem der Ansprüche 9 bis 17, **dadurch gekennzeichnet, dass** mindestens 2 bis 100 dragierte Schichten vorhanden sind.

19. Kaugummi nach Anspruch 18, **dadurch gekennzeichnet, dass** die einzelnen Schichten das/die gleiche(n) Süßungsmittel aufweisen.

20. Kaugummi nach Anspruch 18, **dadurch gekennzeichnet, dass** die einzelnen Schichten unterschiedliche Süßungsmittel enthalten.

21. Kaugummi nach einem der Ansprüche 18 bis 20, **dadurch gekennzeichnet, dass** die einzelnen Schichten das gleiche Calciumsalz und/oder die gleichen Komposite davon enthalten.

22. Kaugummi nach einem der Ansprüche 18 bis 21, **dadurch gekennzeichnet, dass** die einzelnen Schichten unterschiedliche Calciumsalze und/oder unterschiedliche Komposite davon enthalten,

23. Kaugummi nach einem der vorstehenden Ansprüche, **dadurch gekennzeichnet, dass** er zusätzlich Fluoridsalze enthält.

24. Kaugummi nach einem der vorstehenden Ansprüche, **dadurch gekennzeichnet, dass** er Aromastoffe, Füllstoffe und/oder weitere Hilfsstoffe enthält.

25. Verfahren zur Herstellung eines Kaugummis nach einem der Ansprüche 1 bis 24, umfassend das Herstellen eines Kaugummi-Kerns und das Umhüllen des Kaugummi-Kerns mit mindestens einer Schicht, die ein schwer wasserlösliches Calciumsalz und/oder ein Komposit davon umfasst, wobei das schwer wasserlösliche Calciumsalz eine Teilchengröße kleiner 1000 nm aufweist.

26. Verfahren nach Anspruch 25, wobei der Kaugummi-Kern mittels mindestens eines Hartdragier-Schrittes mit der das Calciumsalz und/oder dessen Komposit enthaltenden Schicht umhüllt wird.

27. Verfahren nach Anspruch 26, wobei der Hartdragier-Schritt das Aufbringen einer Lösung oder Suspension, die mindestens ein Süßungsmittel und das Calciumsalz und/oder ein Komposit davon enthält, und das Trocknen der aufgebrachten Lösung oder Suspension umfasst.

28. Verfahren nach Anspruch 25, wobei der Kaugummi-Kern mittels mindestens eines Weichdragier-Schrittes mit der das Calciumsalz und/oder dessen Komposit enthaltenden Schicht umhüllt wird.

29. Verfahren nach Anspruch 28, wobei der Weichdragier-Schritt das Aufbringen einer Lösung oder Suspension, die mindestens ein Süßungsmittel enthält, und das Bestäuben der aufgebrachten Lösung oder Suspension mit einem Süßungsmittel-Pulver umfasst.

30. Verfahren nach Anspruch 28 oder 29, wobei die aufgebrachte Lösung oder Suspension die Gesamtmenge des Calciumsalzes und/oder von dessen Kompositen oder einen Teil davon enthält.

31. Verfahren nach Anspruch 28 oder 29, wobei das Süßungsmittel-Pulver die Gesamtmenge des Calciumsalzes und/oder von dessen Kompositen oder einen Teil davon enthält.

32. Verfahren nach einem der Ansprüche 25 bis 31, wobei die Hartdragier- oder Weichdragier-Schritte mehrmals wiederholt werden.

33. Verfahren nach einem der Ansprüche 25 bis 32, wobei das Calciumsalz ausgewählt ist aus Fluorapatit, carbonathaltigem nichtstöchiometrischem Apatit, Hydroxylapatit und Fluor-dotierten Hydroxylapatit.

34. Verfahren nach einem der Ansprüche 25 bis 33, wobei das Calciumsalz eine Teilchengröße von 5 bis 300 nm aufweist.

35. Verfahren nach einem der Ansprüche 25 bis 34, wobei die umhüllende Schicht 0.001 bis 5 Gew.-%, vorzugsweise 0.01 bis 2 Gew.-% Calciumsalz und/oder eines Kompositen davon enthält.

## Claims

1. Chewing gum, **characterized in that** it is coated with at least one layer containing poorly water-soluble calcium salt and/or a composite thereof, the poorly water-soluble calcium salt having a particle size of smaller than 1000 nm.

2. Chewing gum as claimed in claim 1, **characterized in that** the calcium salt is selected from fluoroapatite, carbonate-containing non-stoichiometric apatite, hydroxylapatite and fluorine-doped hydroxylapatite.

3. Chewing gum as claimed in claim 2, **characterized in that** the poorly water-soluble calcium salt has a particle size of 5 to 300 nm.

4. Chewing gum as claimed in any of the preceding claims, **characterized in that** the poorly water-soluble calcium salt is present in the form of rodlet-like crystals.

5. Chewing gum as claimed in any of the preceding claims, **characterized in that** the coating layer contains 0.001 to 5% by weight and more particularly 0.01 to 2% by weight calcium salt and/or a composite thereof.

6. Chewing gum as claimed in any of the preceding claims, **characterized in that** the coating layer contains a composite of calcium salt and protein component.

7. Chewing gum as claimed in claim 6, **characterized in that** the protein component is selected from gelatine, casein or hydrolyzates thereof, more particularly gelatine.

8. Chewing gum as claimed in any of the preceding claims, **characterized in that** the calcium salt and/or composite thereof is coated with one or more surface modifiers.

9. Chewing gum as claimed in any of the preceding claims, **characterized in that** the at least one coating layer is a coated layer.

10. Chewing gum as claimed in any of claims 1 to 9, **characterized in that** the chewing gum contains sugar.

11. Chewing gum as claimed in claim 10, **characterized in that** the chewing gum and/or the coating layer contain(s) as sweetener sucrose, invert liquid sugar, invert sugar syrup, glucose, glucose syrup, polydextrose, tagatose, trehalose, trehalulose, maltose, lactose, fructose, leucrose, palatinose, condensed palatinose, hydrogenated condensed palatinose or a mixture thereof.

12. Chewing gum as claimed in claim 10 or 11, **characterized in that** the chewing gum and/or the coating layer contains as an additional sweetener fructo-oligosaccharides, lactitol, sorbitol, xylitol, mannitol, maltitol, erythritol, 6-O-α-D-glucopyranosyl-D-sorbitol (1,6-GPS), 1-O-α-D-glucopyranosyl-D-sorbitol (1,1-GPS), 1-O-α-D-glucopyranosyl-D-mannitol (1,1-GPM) or a mixture thereof.

13. Chewing gum as claimed in any of claims 1 to 9, **characterized in that** the chewing gum is sugar-free.

14. Chewing gum as claimed in claim 13, **characterized in that** the chewing gum and the coating layer contain as sweeteners fructo-oligosaccharides, lactitol, sorbitol, xylitol, mannitol, maltitol, erythritol, 6-O-α-D-glucopyranosyl-D-sorbitol (1,6-GPS), 1-O-α-D-glucopyranosyl-D-sorbitol (1,1-GPS), 1-O-α-D-glucopyranosyl-D-mannitol (1,1-GPM) or a mixture thereof.

15. Chewing gum as claimed in claim 12 or 14, **characterized in that** the mixtures are selected from the group consisting of an equimolar or substantially equimolar mixture of 1,6-GPS and 1,1-GPM (isomalt), a mixture of 1,6-GPS, 1,1-GPS and 1,1-GPM, a 1,6-GPS-enriched mixture of 1,6-GPS and 1,1-GPM with a 1,6-GPS content of 57% by weight to 99% by weight and a 1,1-GPM content of 43% by weight to 1% by weight, a 1,1-GPM-enriched mixture of 1,6-GPS and 1,1-GPM with a 1,6-GPS content of 1% by weight to 43% by weight and a 1,1-GPM content of 57% by weight to 99% by weight and a syrup consisting of a mixture of hydrogenated starch hydrolyzate syrup and isomalt syrup or isomalt powder, the dry matter of the syrup consisting of 7 - 52% (weight/weight) 1,6-GPS, 24.5 - 52% (weight/weight) 1,1-GPM, 0 - 52% (weight/weight) 1,1-GPS, 0 - 13% (weight/weight) sorbitol, 2.8 - 13.8% (weight/weight) maltitol, 1.5 - 4.2% (weight/weight) maltotriitol and 3.0 - 13.5% (weight/weight) higher polyols.

16. Chewing gum as claimed in any of the preceding claims, **characterized in that** the chewing gum and/or the coating layer additionally contain(s) one or more intensive sweeteners.

17. Chewing gum as claimed in claim 16, **characterized in that** the intensive sweetener is cyclamate, saccharin, aspartame, glycyrrhizin, neohesperidin dihydrochalcone, stevioside, thaumatin, monellin, acesulfam, alitam, sucralose or a mixture thereof.

18. Chewing gum as claimed in any of claims 9 to 17, **characterized in that** at least 2 to 100 coated layers are present.

19. Chewing gum as claimed in claim 18, **characterized in that** the individual layers contain the same sweetener(s).

20. Chewing gum as claimed in claim 18, **characterized in that** the individual layers containing different sweeteners.

21. Chewing gum as claimed in any of claims 18 to 20, **characterized in that** the individual layers contain the same calcium salt and/or the same composite thereof.

22. Chewing gum as claimed in any of claims 18 to 21, **characterized in that** the individual layers contain different calcium salts and/or different composites thereof.

23. Chewing gum as claimed in any of the preceding claims, **characterized in that** it additionally contains fluoride salts.

24. Chewing gum as claimed in any of the preceding claims, **characterized in that** it contains flavours, fillers and/or other auxiliaries.

25. A process for the production of the chewing gum claimed in any of claims 1 to 24 comprising the steps of producing a chewing gum core and coating the chewing gum core with at least one layer containing a poorly water-soluble calcium salt and/or a composite thereof, the poorly water-soluble calcium salt having a particle size of smaller than 1,000 nm.

26. A process as claimed in claim 25, **characterized in that** the chewing gum core is coated with the layer containing the calcium salt and/or a composite thereof in at least one hard coating step.

27. A process as claimed in claim 26, **characterized in that** the hard coating step comprises applying a solution or suspension containing at least one sweetener and the calcium salt and/or a composite thereof and drying the solution or suspension applied.

28. A process as claimed in claim 25, **characterized in that** the chewing gum core is coated with the layer containing the calcium salt and/or a composite thereof in at least one soft coating step.

29. A process as claimed in claim 28, **characterized in that** the soft coating step comprises applying a solution or suspension containing at least one sweetener and dusting the solution or suspension applied with a sweetener powder.

30. A process as claimed in claim 28 or 29, **characterized in that** the solution or suspension applied contains all or part of the calcium salt and/or composites thereof.

31. A process as claimed in claim 28 or 29, **characterized in that** the sweetener powder contains all or part of the calcium salt and/or composites thereof.

32. A process as claimed in any of claims 25 to 31, **characterized in that** the hard or soft coating steps are repeated several times.

33. A process as claimed in any of claims 25 to 32, **characterized in that** the calcium salt is selected from fluoroapatite, carbonate-containing non-stoichiometric apatite, hydroxylapatite and fluorine-doped hydroxylapatite.

34. A process as claimed in any of claims 25 to 33, **characterized in that** the calcium salt has a particle size of 5 to 300 nm.

35. A process as claimed in any of claims 25 to 34, **characterized in that** the coating layer contains 0.001 to 5% by weight and preferably 0.01 to 2% by weight calcium salt and/or a composite thereof.

## Revendications

1. Gomme à mâcher **caractérisée en ce qu'**elle est enrobée par au moins une couche, cette couche comprenant un sel de calcium peu soluble dans l'eau et/ou une substance composite de ce sel, le sel de calcium peu soluble dans l'eau présentant une taille des particules inférieure à 1000 nm.

2. Gomme à mâcher selon la revendication 1, **caractérisée en ce que** le sel de calcium est choisi parmi la fluorapatite, l'apatite non stoechiométrique contenant du carbonate, l'hydroxyapatite et l'hydroxyapatite dopée au fluor.

3. Gomme à mâcher selon la revendication 2, **caractérisée en ce que** le sel de calcium peu soluble dans l'eau présente une taille des particules de 5 à 300 nm.

4. Gomme à mâcher selon l'une quelconque des revendications précédentes, **caractérisée en ce que** le sel de calcium peu soluble dans l'eau est constitué de cristaux en forme de baguette.

5. Gomme à mâcher selon l'une quelconque des revendications précédentes, **caractérisée en ce que** la couche d'enrobage contient de 0,001 à 5 % en poids, en particulier de 0,01 à 2 % en poids de sel de calcium et/ou de substances composites de ce sel.

6. Gomme à mâcher selon l'une quelconque des revendications précédentes, **caractérisée en ce que** la couche d'enrobage contient une substance composite formée de sel de calcium et d'un composant protéinique.

7. Gomme à mâcher selon la revendication 6, **caractérisée en ce que** le composant protéinique est choisi parmi la gélatine, la caséine ou leurs produits d'hydrolyse, en particulier la gélatine.

8. Gomme à mâcher selon l'une quelconque des revendications précédentes, **caractérisée en ce que** le sel de calcium et/ou ses substances composites sont enrobés avec un ou plusieurs agents modificateurs de surface.

9. Gomme à mâcher selon l'une quelconque des revendications précédentes, **caractérisée en ce que** la couche d'enrobage est une couche dragéifiée.

10. Gomme à mâcher selon l'une quelconque des revendications 1 à 9, la gomme à mâcher contenant du sucre.

11. Gomme à mâcher selon la revendication 10, la gomme à mâcher et/ou la couche d'enrobage contenant, comme agent édulcorant, du saccharose, du sucre liquide inverti, du sirop de sucre inverti, du glucose, du sirop de glucose, du polydextrose, du tagatose, du tréhalose, du tréhalulose, du maltose, du lactose, du fructose, du leucrose, du palatinose, du palatinose condensé, du palatinose condensé hydrogéné, ou un mélange de ceux-ci.

12. Gomme à mâcher selon la revendication 10 ou 11, la gomme à mâcher et/ou la couche d'enrobage contenant, comme agent édulcorant supplémentaire, des fructo-oligosaccharides, du lactitol, du sorbitol, du xylitol, du mannitol, du maltitol, de l'érythritol, du 6-O-α-D-glucopyranosyl-D-sorbitol (1,6-GPS), du 1-O-α-D-glucopyranosyl-D-sorbitol (1,1-GPS), du 1-O-α-D-glycopyranosyl-D-mannitol (1,1-GPM) ou un mélange de ceux-ci.

13. Gomme à mâcher selon l'une quelconque des revendications 1 à 9, la gomme à mâcher étant dépourvue de sucre.

14. Gomme à mâcher selon la revendication 13, la gomme à mâcher et la couche d'enrobage contenant, comme agent édulcorant, des fructo-oligosaccharides, du lactitol, du sorbitol, du xylitol, du mannitol, dua maltitol, de l'érythritol, du 6-O-α-D-glucopyranosyl-D-sorbitol (1,6-GPS), du 1-O-α-D-glucopyranosyl-D-sorbitol (1,1-GPS), du 1-O-α-D-glycopyranosyl-D-mannitol (1,1-GPM) ou un mélange de ceux-ci.

15. Gomme à mâcher selon la revendication 12 ou 14, les mélanges étant choisis dans le groupe constitué par un mélange équimolaire ou presque équimolaire de 1,6-GPS et de 1,1-GPM (isomalte), un mélange de 1,6-GPS, de 1,1-GPS et de 1,1-GPM, un mélange enrichi en 1,6-GPS, de 1,6-GPS et de 1,1-GPM avec une teneur en 1,6-GPS de 57 % en poids à 99 % en poids et une teneur en 1,1-GPM de 43 % en poids à 1 % en poids, un mélange enrichi en 1,1-GPM, de 1,6-GPS et de 1,1-GPM avec une teneur en 1,6-GPS de 1 % en poids à 43 % en poids et une teneur en 1,1-GPM de 57 % en poids à 99 % en poids, et un sirop, constitué par un mélange de sirop d'hydrolysat d'amidon hydrogéné et de sirop d'isomalte ou de poudre d'isomalte, la teneur en substance sèche du sirop étant constituée par 7-52 % (p/p) de 1,6-GPS, 24,5-52 % (p/p) 1,1-GPM, 0-52 % (p/p) 1,1-GPS, 0-13 % (p/p) de sorbitol, 2,8-13,8 % (p/p) de maltitol, 1,5-4,2 % (p/p) de maltotriitol et 3,0-13,5 % (p/p) de polyols supérieurs.

16. Gomme à mâcher selon l'une quelconque des revendications précédentes, la gomme à mâcher et/ou la couche d'enrobage contenant en outre un ou plusieurs édulcorants intenses.

17. Gomme à mâcher selon la revendication 16, l'édulcorant intense étant le cyclamate, la saccharine, l'aspartame, la glycyrrhizine, la néohespéridine-dihydrochalcone, le stévioside, la thaumatine, la monelline, l'acésulfame, l'alitame, le sucralose ou un de leurs mélanges.

18. Gomme à mâcher selon l'une quelconque des revendications 9 à 17, **caractérisée en ce qu'**elle comprend au moins 2 à 100 couches dragéifiées.

19. Gomme à mâcher selon la revendication 18, **caractérisée en ce que** les couches individuelles comprennent le ou les même(s) édulcorant(s).

20. Gomme à mâcher selon la revendication 18, **caractérisée en ce que** les couches individuelles comprennent des édulcorants différents.

21. Gomme à mâcher selon l'une quelconque des revendications 18 à 20, **caractérisée en ce que** les couches individuelles comprennent le même sel de calcium et/ou les mêmes substances composites de ce sel.

22. Gomme à mâcher selon l'une quelconque des revendications 18 à 21, **caractérisée en ce que** les couches individuelles comprennent des sels de calcium différents et/ou des substances composites différentes de ces sels.

23. Gomme à mâcher selon l'une quelconque des revendications précédentes, **caractérisée en ce qu'**elle contient en outre des sels fluorures.

24. Gomme à mâcher selon l'une quelconque des revendications précédentes, **caractérisée en ce qu'**elle contient des substances aromatisantes, des charges et/ou d'autres adjuvants.

25. Procédé de préparation d'une gomme à mâcher selon l'une quelconque des revendications 1 à 24, comprenant la préparation d'un noyau de gomme à mâcher et l'opération d'enrobage du noyau de gomme à mâcher avec au moins une couche qui comprend un sel de calcium peu soluble dans l'eau et/ou une substance composite de ce sel, le sel de calcium peu soluble dans l'eau présentant une taille des particules inférieure à 1000 nm.

26. Procédé selon la revendication 25, dans lequel le noyau de gomme à mâcher est enrobé avec la couche contenant le sel de calcium et/ou une substance composite de ce sel au moyen d'au moins une étape de dragéification dure.

27. Procédé selon la revendication 26, dans lequel l'étape de dragéification dure comprend l'application d'une solution ou suspension qui contient au moins un édulcorant et le sel de calcium et/ou une substance composite de ce sel, et le séchage de la solution ou suspension appliquée.

28. Procédé selon la revendication 25, dans lequel le noyau de gomme à mâcher est enrobé avec la couche contenant le sel de calcium et/ou une substance composite de ce sel au moyen d'au moins une étape de dragéification molle.

29. Procédé selon la revendication 28, dans lequel l'étape de dragéification molle comprend l'application d'une solution ou suspension qui contient au moins un édulcorant, et le saupoudrage de la solution ou suspension appliquée avec un édulcorant en poudre.

30. Procédé selon la revendication 28 ou 29, dans lequel la solution ou suspension appliquée contient la quantité totale de sel de calcium et/ou des substances composites de ce sel ou une partie de cette quantité.

31. Procédé selon la revendication 28 ou 29, dans lequel l'édulcorant en poudre contient la quantité totale de sel de calcium et/ou des substances composites de ce sel ou une partie de cette quantité.

32. Procédé selon l'une quelconque des revendications 25 à 31, dans lequel l'étape de dragéification dure ou molle est répétée plusieurs fois.

33. Procédé selon l'une quelconque des revendications 25 à 32, dans lequel le sel de calcium est choisi parmi la fluorapatite, l'apatite non stoechiométrique contenant du carbonate, l'hydroxyapatite et l'hydroxyapatite dopée au fluor.

34. Procédé selon l'une quelconque des revendications 25 à 33, dans lequel le sel de calcium présente une taille de particules de 5 à 300 nm.

35. Procédé selon l'une quelconque des revendications 25 à 34, dans lequel la couche d'enrobage contient de 0,001 à 5 % en poids, de préférence de 0,01 à 2 % en poids de sel de calcium et/ou d'une substance composite de ce sel.
